(19) Europäisches Patentamt
European Patent Office
Office européen des brevets

(11) **EP 2 346 386 B1**

(12) **EUROPÄISCHE PATENTSCHRIFT**

(45) Veröffentlichungstag und Bekanntmachung des Hinweises auf die Patenterteilung:
**10.04.2013  Patentblatt 2013/15**

(21) Anmeldenummer: **09777799.9**

(22) Anmeldetag: **11.08.2009**

(51) Int Cl.:
***A61B 3/10*** *(2006.01)*

(86) Internationale Anmeldenummer:
**PCT/EP2009/005811**

(87) Internationale Veröffentlichungsnummer:
**WO 2010/017954 (18.02.2010 Gazette 2010/07)**

(54) **TIEFENAUFLÖSENDE OPTISCHE KOHÄRENZREFLEKTOMETRIE**

OPTICAL COHERENCE REFLECTOMETRY WITH DEPTH RESOLUTION

RÉFLECTOMÉTRIE OPTIQUE COHÉRENTE À RÉSOLUTION EN PROFONDEUR

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **12.08.2008  AT 12502008
10.10.2008  DE 102008051272**

(43) Veröffentlichungstag der Anmeldung:
**27.07.2011  Patentblatt 2011/30**

(73) Patentinhaber: **Carl Zeiss Meditec AG
07745 Jena (DE)**

(72) Erfinder:
• **HACKER, Martin
07745 Jena (DE)**
• **BARTH, Roland
07743 Jena (DE)**
• **BERGNER, Roland
07745 Jena (DE)**
• **BISSMANN, Wilfried
07749 Jena (DE)**
• **VON BÜNAU, Rudolf
07749 Jena (DE)**
• **EBERSBACH, Ralf
04626 Schmölln (DE)**
• **KOSCHMIEDER, Ingo
07743 Jena (DE)**
• **FERCHER, Adolf, Friedrich
A-1230 Wien (AT)**
• **GRAJCIAR, Branislav
321 08 Bratislava (SK)**

(74) Vertreter: **Geyer, Fehners & Partner
Patentanwälte
Perhamerstrasse 31
80687 München (DE)**

(56) Entgegenhaltungen:
**WO-A-97/30627          WO-A-2007/065670
WO-A-2007/100935**

• **JOHANNES F DE BOER ET AL: "Polarization Effects in Optical Coherence Tomography of Various Biological Tissues" IEEE JOURNAL OF SELECTED TOPICS IN QUANTUM ELECTRONICS, IEEE SERVICE CENTER, PISCATAWAY, NJ, US, Bd. 5, Nr. 4, 1. August 1999 (1999-08-01), XP011062584 ISSN: 1077-260X**
• **J.M. SCHMITT, S.H. XIANG, K.M. YUNG: "Speckle in optical coherence tomography" JOURNAL OF BIOMEDICAL OPTICS, Bd. 4, Nr. 1, Januar 1999 (1999-01), Seiten 95-105, XP002556340**
• **T.M. JORGENSEN, L. THRANE, M. MOGENSEN, F. PEDERSEN, P.E. ANDERSEN: "Speckle reduction in optical coherence tomography images of human skin by a spatial diversity method" PROC. OF SPIE OSA BIOMEDICAL OPTICS, Bd. 6627, 2007, XP002556341**

**Beschreibung**

Die Erfindung bezieht sich auf eine Vorrichtung zur Messung an einem Auge, insbesondere zur Messung von okulären Distanzen wie Vorderkammertiefe, Linsendicke, Homhautdicke oder Achslänge, wobei die Vorrichtung ein Interferometer umfaßt, mindestens einen Meßstrahl entlang einer optischen Achse in das Auge fokussiert, rückgestreute Strahlung aufnimmt und durch Zeitdomänen-, Spektraldomänen- oder Fourierdomänen-Kohärenzreflektrometrie interferometrisch ein Strukturen des Auges anzeigendes Meßsignal erzeugt, und eine Verstelleinrichtung zur lateralen und/oder axialen Verschiebung des Fokus im Auge oder zur Variation eines Polarisationszustandes des Meßstrahls und eine Steuereinrichtung, die das Interferometer ansteuert, aufweist. Die Erfindung bezieht sich weiter auf ein Verfahren zur Messung an einem Auge, insbesondere zur Messung von okulären Distanzen wie Vorderkammertiefe, Linsendicke, Homhautdicke oder Achslänge, wobei mindestens ein Meßstrahl entlang einer optischen Achse in das Auge fokussiert, rückgestreute Strahlung aufgenommen und durch Zeitdomänen-, Spektraldomänen- oder Fourierdomänen-Kohärenzreflektrometrie interferometrisch ein Strukturen des Auges anzeigendes Meßsignal erzeugt wird und die Lage des Fokus im Auge lateral und/oder axial verschoben oder ein Polarisationszustand des Meßstrahls variiert wird.

Axiale Augenlängen und intraokuläre Distanzen werden heute mittels optischer KurzkohärenzInterferometrie (KKI) gemessen. Optische Kurzkohärenz-Interferometrie hat gegenüber der früher dominierenden Ultraschall-Methode die Vorteile der berührungsfreien und hochpräzisen Arbeitsweise. Das Auge befindet sich hierbei im Messarm eines Kurzkohärenz-Interferometers, das ist beispielsweise ein Zweistrahl-Interferometer, das mit Licht kurzer Kohärenzlänge beleuchtet wird. Ein (Kurzkohärenz-)Interferogramm tritt am Interferometer Ausgang hierbei nur auf, wenn sich eine Licht reflektierende oder zurück streuende Struktur im Messarm bis auf die Kohärenzlänge genau im selben optischen Abstand vom Strahlteiler befindet, wie der Referenzspiegel; dieser Bereich wird häufig als "Kohärenzfenster" bezeichnet. Das Kohärenzfenster hat eine Ausdehnung in Strahlrichtung von der Größe der Kohärenzlänge, die bei Kurzkohärenzverfahren meist nur einige Mikrometer beträgt. Bei dem sogenannten "Kurzkohärenz-Scan" wird das Auge durch Verschieben des Referenzspiegels von dem Kohärenzfenster in Strahlrichtung entlang der Sehachse (z-Richtung oder Objekttiefe) abgetastet. Wenn sich eine lichtremittierende Stelle des Auges in dem Kohärenzfenster befindet, tritt am Interferometerausgang, wie schon gesagt, ein Kurzkohärenz-Interferogramm auf. Die hierzu erforderliche Bewegung des Referenzspiegels stellt den Messvorgang dar; die zu messenden Abstände von Grenzflächen werden am Interferometerausgang durch die den betreffenden Grenzflächen zugeordneten Kurzkohärenz-Interferogramme entlang des Referenzspiegelweges z markiert. Diese Abfolge von Kurzkohärenz-Interferogrammen mit der z-abhängigen Rückstreuintensität *I(z)* heißt in Analogie zu ähnlichen Verfahren in der Ultraschall-Technik "A-Scan" Signal.

Die optische Kohärenz-Domain-Reflektometrie (OCDR), oder auch Kurzkohärenzinterferometrie (KKI) genannt, dient dazu, Ort und Größe von Streuzentren innerhalb einer Probe, z. B. dem menschlichen Auge, zu erfassen. Für einen Überblick über entsprechende Literatur zur optischen Kohärenz-Domain-Reflektometrie sei auf die US 2006/0109477 A1 verwiesen. Diese Patentanmeldung schildert auch das Grundprinzip der bildgebenden optischen Kohärenztomographie (OCT). Für die OCDR sind die Varianten Zeit-Domain OCDR (timedomain oder TD-OCDR) mit zum Scannen weglängenverstelltem Referenzarm und Fourier-Domaine OCDR (FD-OCDR / FD-KKI) mit festem Referenzarm und Auswertung spektraler Information bekannt. Letztere unterscheidet man nochmals in eine Variante unter Verwendung breitbandiger Lichtquellen und spektrometerbasierter Detektion (spectral domain oder SD-OCDR) und in eine Variante unter Verwendung spektral durchstimmbarer Lichtquellen und breitbandiger Detektoren (swept-source oder SS-OCDR).

Bei der empfindlicheren Fourier-Domain Kurzkohärenz-Interferometrie (FD-KKI) wird das am Interferometerausgang austretende Licht von einem Spektrometer analysiert; mit Hilfe einer Fourier-Transformation (FT) erhält man unter geeigneten Bedingungen, die zum Stand der Technik gehören (US 7330270 B2), aus dem gemessenen Intensitäts-Spektrogramm I(k) das A-Scan Signal in z-Richtung entlang dem beleuchtenden Messstrahl rekonstruiert:

$$r(z) = FT\{I(k)\} \tag{1}$$

mit der Wellenzahl $k = \dfrac{2\pi}{\lambda} n$ , c ist die Lichtgeschwindigkeit, $\lambda$ die Wellenlänge, n der jeweiligen Brechungsindex und r(z) der Streuamplitude, deren Betragsquadrat der Streuintensität I(z) entspricht.

Problematisch für Kurzkohärenzinterformetrie in Form der FD-OCDR und FD-OCT, ist die feste Verknüpfung von Meßbereich und Meßauflösung. Der Stand der Technik kennt viele Druckschriften, die sich mit der Vermessung von Objekten in Bereichen umfaßt, die geometrisch gegenüber der gewünschten Auflösung um mehrere Größenordnungen größer sind. Ein Beispiel für eine solche Meßaufgabe ist die Vermessung von Bereichen am menschlichen Auge, z.B. die Erfassung von Strukturen sowohl im Vorderbereich des Auges, beispielsweise an der Hornhaut, als auch an der Retina.

Bei OCDR sind die axiale und die laterale Auflösung weitgehend entkoppelt. Die axiale Auflösung ist im Wesentlichen durch die Kohärenzlänge der Quelle gegeben, also umgekehrt proportional zur Gesamtbreite des in der Interferometeranordnung verwendeten Spektrums. In lateraler Richtung ist die erreichbare Auflösung durch die laterale Ausdehnung des Fokus bzw. der im Fokusbereich gegebenen Strahltaille gegeben. Das Streusignal eines Ortes ist somit die Überlagerung der aus dem kleinsten auflösbaren Volumen rückgestreuten Strahlung.

Eine Anwendung, die für die OCDR-Technik von besonderem Interesse ist, ist die Streckenmessung im Auge. Bekannte interferometrisch arbeitende Geräte ermöglichen derzeit entweder eine befriedigende Achslängenmessung oder eine Teilstreckenmessung in der Vorderkammer, z. B. Erfassung der Vorderkammertiefe und der Linsendicke. Bei den einzelnen Teilstreckenmessungen muß dabei der Meßstrahl sehr genau im Auge justiert werden.

Bekannte Verfahren zu Teilstrecken- oder Achslängenmessung arbeiten entlang der Sehachse eines Auges (z. B. Lexer et al., "Wavelength-tuning interferometry of intraocular distances", APPLIED OPTICS, Vol. 36, No. 25). Sie nützen somit im Allgemeinen nicht die auf der optischen Achse des Auges detektierbaren, starken und klar definierten spekularen Reflexe von Grenzflächen im Auge für Teilstreckenmessungen, sondern verwerten Signale aus Volumenstreuungen in Strukturen des Auges.

Ein entspredendes Gerät und Verfahren ist aus der WO 2007/065670 A bekannt, die Meshunale der Präambel der Ansprüche 1 und 7 offenbat.

Die Veröffentlichung T. M. Jorgensen, L. Thrane, M. Mogensen, F. Pedersen, P. E. Andersen: "Speckle reduction in optical coherence tomography images of human skin by a spatial diversity method", Proc. of SPIE-OSA Biomedical Optics, Bd. 6627, 2007, XP002556341, befaßt sich mit der Reduzierung von Speckle-Einflüssen bei der Erzeugung von OCT-Bildern, bei denen es sich B-Scan-Bilder handelt. Dabei wird für jedes B-Scan-Bild, das bekanntermaßen aus einer Serie von A-Scan-Bildern erzeugt wird, eine eigene axiale Fokuslage verwendet. Die so bei verschiedenen Fokuseinstellungen erhaltenen B-Scan-Bilder werden dann gemittelt, um Speckle-Effekte zu mindern.

Die WO 2007/100935 A2 zielt auf eine Verbesserung von OCT-Bildern, in denen eine Vorrichtung zur Modifikation der Wellenfronten des Lichts, das von der Probe zurückkehrt, verwendet wird. Zur Speckle-Reduzierung ist dabei vorgeschlagen, die Wellenfront-Modifikation für aufeinanderfolgende Scans entlang oder quer zur optischen Achse so einzustellen, daß die jeweiligen Scans unterschiedliche Speckle-Muster enthalten. Eine Mittelung führt dann zu einem reduzierten Speckle-Kontrast ohne Strukturverlust im erzeugten Bild. Weiter ist in der WO-Schrift erwähnt, daß zur Detektion von Sub-Strukturen innerhalb des Fokalvolumens einer Probe der Fokus des einfallenden Strahls modifiziert werden kann, indem der Fokus auf kleine Sub-Strukturen im Fokalvolumen der Probe abgestimmt wird, beispielsweise um eine maximale Reflexion für Sub-Strukturen bestimmter Größe und in bestimmtem Abstand zueinander erreicht wird. Ein Vergleich der Messungen mit derart gemustertem Fokus mit Messungen, die ein anderes Fokusmuster aufweisen, kann so zu Informationen über Strukturen führen, die kleiner sind, als das Fokalvolumen.

Aus der Veröffentlichung Johannes F. De Boer et al.: "Polarization Effects in Optical Coherence Tomography of Various Biological Tissues", IEEE Journal of Selected Topics in Quantum Electronics, IEEE Service Center, Piscataway, NJ, US, Bd. 5, Nr. 4, 1. August 1999 (1999-08-01), XP011062584, ISSN: 1077-260X, ist es bekannt, zum Mindern von Speckle-Effekten bei OCT-Bildern Bilder zu summieren, die aus Bildkanälen stammen, deren Polarisation orthogonal zueinander ist.

Zur Gewinnung optimaler Signale gibt es einige Erfordernisse, die bei in vivo Messungen am Auge erfüllt sein müssen und teilweise für beide Kurzkohärenz-Interferometrie Methoden (FD-OCDR bzw. TD-OCDR) gelten. Hierzu gehören:

1. Intensitätsverhältnis Messstrahl zu Referenzstrahl anpassen.
2. Mehrfache Referenzstrahlen wegen begrenzter Feldtiefe (FD-KKI).
3. Kurze Messzeiten.
4. Intensitätsanpassung an Reflektivitäten der Augenstrukturen.
5. Identifizierbarkeit der den registrierten Signalen zuzuordnenden Augenstruktur.

Zu 1. Das gilt für TD-KKI und FD-KKI zur Optimierung der Sensitivität.

Zu 2. Die Fourier-Domain Kurzkohärenz-Interferometrie hat - bei heute gängigen Detektor-Arrays - eine auf wenige Millimeter begrenzte Messfeldtiefe T

$$T = N \cdot \pi / \Delta K \qquad (2)$$

Mit N = gleich der Anzahl der Abtastpunkte (- Anzahl der Detektor-Array Elemente in $\lambda$-Richtung); $\Delta K$ ist die spektrale Breite $\Delta\lambda$ des Messlichts, ausgedrückt als Breite des Streuvektors K = 4 $\cdot$ $\pi/\lambda$. Das reicht in der Regel zur Corneadickenmessung und, je nach spektraler Breite des Messlichts, auch zur Messung der Vorderkammer-Tiefe.

Augenlängenmessungen sind mit den heute kommerziell erhältlichen Arrays nur bei sehr geringer Tiefenauflösung (kleine spektrale Breite des Messlichts) möglich. Daher müssen bei der FD-KKI 2 Messfelder in unterschiedlicher Tiefe mit Hilfe zweier Referenzstrahlen oder zweier Probenstrahlen realisiert werden.

Zu 3. Zur Augenlängenmessung müssen wegen der Eigenbewegung des (lebenden) Auges zur zweifelsfreien Distanzmessung jedoch beide Kurzkohärenz-Interferogramme, die die zu messende Strecke markieren, gleichzeitig oder sehr zeitnah registriert werden, was im Falle der FD-KKI mit der auf wenige Millimeter beschränkten Feldtiefe nicht ohne weiteres geht; denn man müsste erst den die Position des Messfelds definierenden Referenzspiegel verschieben, um auch das zweite Signal sehen zu können. Ein Ausweg besteht darin, mittels eines zweiten Referenzstrahls ein zweites Messfeld in der erforderlichen Tiefenposition zu realisieren. Man erhält so FD-A-Scan Signalpaare, aus deren KK-Interferogrammen, unter Berücksichtigung der Wegdifferenz der Referenzstrahlen, die betreffende Distanz bestimmbar ist.

Bei der TD-KKI wiederum können die 2 Messpositionen wegen der erforderlichen Bewegung von Referenzspiegeln nicht simultan erfasst werden. Um nun den Zeitabstand zwischen den beiden Messpositionen auch hier zu verringern, kann man auch hier mit 2 Referenzstrahlen arbeiten, die 2 Kohärenzfenster realisieren. Zur Gewinnung mehrerer seitlich versetzter A-Scan Signale kann man, wie üblich, den Messstrahl mittels einer Spiegelvorrichtung seitlich verschieben. Viel schneller jedoch werden transversal zueinander versetzte A-Scan Signale der für die Längenmessung relevanten Augenstrukturen mittels Parallel-KKI gewonnen, wobei auf Zylinderlinsen basierende anamorphotische Optiken den Messstrahl in die Ebenen der relevanten Augenstrukturen linienförmig fokussieren und die transversal benachbarten A-Scan Signale innerhalb dieses Linienfokus durch das Detektorarray am Interferometerausgang selektieren.

Zu 4. Intensitätsanpassung an Reflektivitäten der Augenstrukturen. Die Reflektivität der Comea ist rund $10^3$ x größer als jene einzelner Retinaschichten. Damit Reflexe der Retinaschichten im A-Scan Signal im Vergleich zum Comeasignal nicht untergehen, ist es sinnvoll, diese Strukturen mit größerer Strahlintensität zu beleuchten als die Comea.

Zu 5. Bei beiden Kurzkohärenz-Interferometrie Methoden erfolgt der A-Scan etwa entlang der Sehachse durch das Auge. Hierbei treten an markanten Gewebegrenzen, wie der Comea-Vorderfläche und den Fundusschichten, Lichtreflexe auf, die die Basis für die Distanzmessung bilden. Es treten jedoch auch Lichtreflexe und Messsignale an Strukturen auf, die nicht richtig identiflzierbar sind; Fehlmessungen sind das Ergebnis.

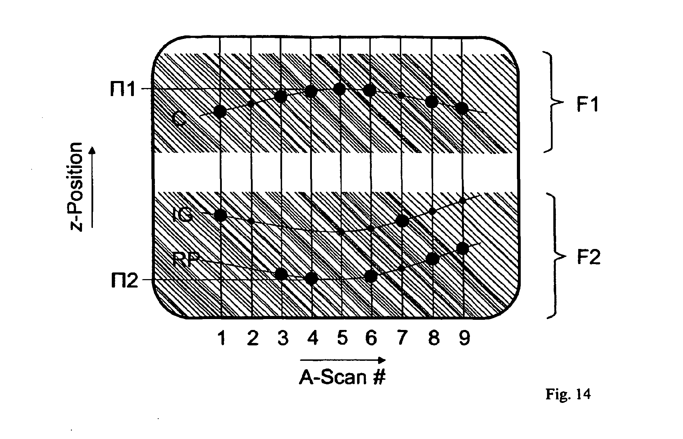

Figur 14 zeigt Beispiele hierzu:

A-Scan #1 zeigt im Messfenster F1 ein starkes Signal an der Comea (C), im Messfenster F2 ein starkes Signal an der inneren Grenzmembran (IG) und kein Signal vom retinalen Pigmentepithel (RP): es kommt zu einer Fehlmessung.
A-Scan #2 zeigt ein schwaches Signal an der Comea (C), ein schwaches Signal an der inneren Grenzmembran (IG) und kein Signal vom retinalen Pigmentepithel (RP): es kommt zu einer Fehlmessung.
A-Scan #3 & 4 zeigen ein starkes Signal an der Cornea (C), kein Signal an der inneren Grenzmembran (IG) und ein starkes Signal vom retinalen Pigmentepithel (RP): es kommt zu einer richtigen Messung.
A-Scan #5 zeigt ein starkes Signal an der Cornea (C), ein schwaches Signal an der inneren Grenzmembran (IG) und kein Signal vom retinalen Pigmentepithel (RP): es kann zu einer Fehlmessung kommen.
A-Scan #6, 8 & 9 zeigen ein starkes Signal an der Comea (C), ein schwaches Signal an der inneren Grenzmembran (IG) und starkes Signal vom retinalen Pigmentepithel (RP): es kommt zu einer richtigen Messung.
A-Scan #7 zeigt ein schwaches Signal an der Comea (C), ein starkes Signal an der inneren Grenzmembran (IG) und ein schwaches Signal vom retinalen Pigmentepithel (RP): es kommt wahrscheinlich zu einer Fehlmessung.

Der Erfindung liegt deshalb die Aufgabe zugrunde, mittels Zeitdomänen-Spektraldomänen oder Fourierdomänen-Kohärenzreflektrometrie ein Auge und insbesondere eine Streckenlänge verbessert erfassen zu können. Besonders bevorzugt soll gleichzeitig eine Achslängenmessung und eine weitere Teilstreckenmessung am Auge erfolgen. Das Signal/Rausch-Verhältnis und insbesondere das Vermögen zur Grenzftächenbestimmung auf Basis von Volumenstreuungssignalen sollen verbessert, Meßfehler vermieden und die Anforderungen an die Justage des Auges reduziert werden.

Diese Aufgabe wird erfindungsgemäß gelöst mit einer Vorrichtung nach Auspruch 1 zur Messung an einem Auge, insbesondere zur Messung von Vorderkammertiefe. Linsendicke, Hornhautdicke oder Achslänge, sowie von Netzhautschichtdicken, wie beispielsweise der Nervenfaserschichtdicke oder dem Abstand zwischen innerer Grenzmembran (ILM) und retinalem Pigmentepithelium (RPE), wobei die Vorrichtung ein Interferometer umfaßt, mindestens einen Meßstrahl entlang einer optischen Achse in das Auge fokussiert, rückgestreute Strahlung aufnimmt und durch Zeitdomänen-, Spektraldomänen- oder Fourierdomänen-Kohärenzreflektrometrie interferometrisch ein Strukturen des Auges anzeigendes Meßsignal erzeugt und eine Verstelleinrichtung zur lateralen und/oder axialen Verschiebung des Fokus im Auge oder zur Variation eines Polarisationszustandes des Meßstrahls und eine Steuereinrichtung, die das Interferometer ansteuert, aufweist, wobei die Steuereinrichtung aus der rückgestreuten Strahlung mehrere A-Scan-Einzelsignale erzeugt und diese zu einem A-Scan-Meßsignal zusammenfaßt und so ausgebildet ist, daß sie die Verstelleinrichtung zur Verschiebung der Lage des Fokus oder zur Polarisationsvariation während der Aufnahme der rückgestreuten Strahlung, aus der die Steuereinrichtung die A-Scan-Einzelsignale erzeugt, ansteuert und wobei rückgestreute Strahlung bei mehreren verschiedenen Lagen des Fokus oder mehreren verschiedenen Polarisationszuständen der Meßstrahlung zum A-Scan-Meßsignal beiträgt.

Die Aufgabe wird erfindungsgemäß weiter gelöst mit einem Verfahren nach Ausspruch 7 zur Messung an einem Auge, insbesondere zur Messung von Vorderkammertiefe, Linsendicke, Homhautdicke oder Achslänge, wobei mindestens ein Meßstrahl entlang einer optischen Achse in das Auge fokussiert, rückgestreuten Strahlung aufgenommen und durch Zeitdomänen-, Spektraldomänen- oder Fourierdomänen-Kohärenzreflektrometrie interferometrisch ein Strukturen des Auges anzeigendes Meßsignal erzeugt wird und wobei die Lage des Fokus im Auge lateral und/oder axial verschoben oder ein Polarisationszustand des Meßstrahls variiert wird, wobei aus der rückgestreuten Strahlung interferometrisch mehrere A-Scan-Einzelsignale erzeugt und zu einem A-Scan-Meßsignal zusammengefaßt werden, wobei die Verschiebung der Lage des Fokus oder die Variation des Polarisationszustandes während der Aufnahme der rückgestreuten Strahlung, aus der die mehreren A-Scan-Einzelsignale erzeugt werden, ausgeführt wird und wobei rückgestreute Strahlung bei mehreren verschiedenen Lagen des Fokus oder mehreren verschiedenen Polarisationszuständen der Meßstrahlung zum A-Scan-Meßsignal beiträgt.

Weitebildungen betreffen Summen-A-Scan Signale, die als Summe aus mehreren seitlich zueinander versetzten einfachen A-Scan Signalen oder als Summe aus mehreren zeitlich aufeinander folgenden einfachen A-Scans an derselben Stelle oder als Summe aus mehreren seitlich zueinander versetzten und zeitlich aufeinander folgenden einfachen A-Scan Signalen oder als Summe aus mehreren innerhalb einer Fläche seitlich zueinander versetzten einfachen A-Scan Signalen oder als Summe aus mehreren innerhalb einer Fläche seitlich zueinander versetzten und zeitlich aufeinander folgenden einfachen A-Scan Signalen oder als Summe aus mehreren einfachen A-Scan Signalen aus bestimmten transversalen Positionen im Auge sowie zu bestimmten Zeitpunkten innerhalb der Herzpulsperiode gewonnen werden.

Oft entspricht bei OCDR-Interferometem der Fokus der Meßstrahlung auch dem Gebiet, insbesondere dem Fokus, aus dem die rückgestreuten Strahlung aufgenommen wird.

Die Erfindung erreicht ein verbessertes Signal/Rausch-Verhältnis für das A-Scan-Meßsignal dadurch, daß dieses aus mehreren A-Scan-Einzelsignalen zusammengesetzt wird, wobei die Fokuslage und/oder der Polarisationszustand der Meßstrahlung während der Aufnahme der Strahlung für die A-Scan-Einzelsignale verändert wird. Somit unterscheiden sich die A-Scan-Einzelsignale hinsichtlich der Fokuslage bzw. des Polarisationszustandes der Meßstrahlung.

Erfindungsgemäß trägt also zum A-Scan-Meßsignal rückgestreuten Strahlung bei, die an verschiedenen Fokuslagen bzw. bei verschiedenen Polarisationszuständen der Meßstrahlung gewonnen wurde. Die Vorrichtung bzw. das Verfahren erzeugen also ein A-Scan-Meßsignal, das Informationen über Rückstreustärke und Ort von rückstreuenden Strukturen des Auges enthält, wobei die Ortsangabe in Tiefenrichtung gegeben ist. Der Begriff A-Scan ist dabei auf in der Ophthalmologie übliche Weise zu verstehen. Der A-Scan liefert Daten längs des Auges, d. h. von posterior nach anterior. Im Sinne der vorliegenden Erfindung ist ein A-Scan-Signal ein Signal, das die Rückstreuintensität von Strukturen des Auges längs der Tiefenrichtung des Auges wiedergibt.

Die Erfindung kombiniert nun einen Satz von mehreren A-Scan-Einzelsignalen zu einem einzigen A-Scan-Meßsignal, wobei sich die A-Scan-Einzelsignale des Satzes hinsichtlich der Lage des Fokus bzw. des Polarisationszustandes der Meßstrahlung, welche zur Gewinnung des A-Scan-Einzelsignals eingestrahlt wurde, voneinander unterscheiden. Im Ergebnis erreicht die Erfindung ein einziges A-Scan-Meßsignal, das aus Meßstrahlung erzeugt wurde, die ein Gemisch verschiedener Fokuslagen oder Polarisationszustände aufweist. Die Fokuslagen- bzw. Polarisationszustandsveränderung muß dabei nicht zwingend in vollem Veränderungsumfang während der Aufnahme der rückgestreuten Strahlung für eines der mehreren A-Scan-Einzelsignale ausgeführt werden. Vielmehr ist es zweckmäßig den vollen Veränderungsumfang über einen oder mehrere Sätze zu verteilen.

Die Variation der Fokuslage bzw. des Polarisationszustandes der Meßstrahlung während der Aufnahme rückgestreuter Strahlung, die für die Erzeugung der A-Scan-Einzelsignale verwertet wird, muß weiter nicht synchronisiert zur Erzeugung der A-Scan-Einzelsignale erfolgen. Hier liegt neben der Tatsache, daß mehrere A-Scan-Einzelsignale zu einem gemeinsamen A-Scan-Meßsignal zusammengefaßt werden, ein weiterer Unterschied zu üblichen bildgebenden Verfahren, die zwingend auf eine Synchronisierung zwischen Fokuslagenverstellung und Signalerzeugung angewiesen sind. Die in einer bevorzugten Variante der Erfindung nicht vorhandene Synchronisierung zwischen Fokuslagen- bzw. Polarisationsvariation und Erzeugung der A-Scan-Einzelsignale zeigt sich durch eine Variation der Phase zwischen der Fokuslagen- bzw. Polarisationsvariation und der Aufnahme der rückgestreuten Strahlung zur Erzeugung von A-Scan-Einzelsignalen. Es ist keine starre Phasenbeziehung zwischen diesen beiden Vorgängen gegeben; vielmehr variiert die Phase. Besonders deutlich wird dies in der erwähnten Variante dadurch, daß die Phasenlage zu Beginn der Aufnahme von Satz der mehreren A-Scan-Einzelsignalen, die dann zu dem einen A-Scan-Meßsignal zusammengefaßt werden, zu Satz (d. h. für verschieden Sätze von A-Scan-Einzelsignalen) variiert. Erzeugt die Vorrichtung bzw. das Verfahren also nacheinander mehrere A-Scan-Meßsignale, wobei jedes A-Scan-Meßsignal aus einem Satz nacheinander aufgenommener A-Scan-Einzelsignale erzeugt wird, liegt zum Beginn eines jeden Satzes nicht dieselbe Phase zur Variation der Fokuslage bzw. des Polarisationszustandes vor. Diese vorteilhafte Erfindungseigenschaft erlaubt die Verwendung eines einfachen Aufbaus zur Variation der Fokuslage bzw. des Polarisationszustandes, da keine Rückkopplung zwischen der Variation und der Aufnahme der rückgestreuten Strahlung für die A-Scan-Einzelsignale erforderlich ist. Die Variation kann beispielsweise mittels eines freilaufenden Oszillators vorgenommen werden, und eine ständige Messung bzw. Ermittlung der aktuellen Fokuslage bzw. des aktuellen Polarisationszustandes entfällt bzw. wird im Verfahren oder von der Steuereinrichtung nicht ausgeführt.

Die Efinder erkannten, dass eine Verbesserung des Signal/Rausch-Verhältnisses und die Vermeidung von Meßfehlern des A-Scan-Meßsignals vorzugsweise erreicht wird, ohne daß die aktuelle Fokusverschiebung bzw. Polarisationsvariation der Meßstrahlung berücksichtigt wird, was zu einem erheblich vereinfachten Aufbau bzw. ein erheblich vereinfachtes Verfahren führt. Die Fokusverschiebung bzw. Polarisationsvariation geht vorzugsweise bei der Zusammenfassung der A-Scan-Einzelsignale zum A-Scan-Meßsignal nicht ein. Um den apparativen Aufwand gering zu halten, erfolgt also nicht unbedingt eine Bildgebung.

Das erfindungsgemäße Konzept erlaubt es auf einfache Art und Weise, verschiedene Effekte, welche das Signal/Rausch-Verhältnis mindern oder Meßfehler erzeugen können, zu unterdrücken. Die Erfindung kann deshalb besonders vorteilhaft weitergebildet werden zur Streckenmessung am Auge, da die Strecke begrenzenden Grenzflächen besser erfaßt werden können. Es ist deshalb in einer Weiterbildung der Erfindung vorgesehen, daß die Steuereinrichtung eine Streckenmessung am Auge durchführt. Analoges gilt für das erfindungsgemäße Verfahren.

Das Rückstreusignal eines Ortes im Auge ist bei der erfindungsgemäß verwendeten OCDR durch die Überlagerung der aus der kleinsten auflösbaren Volumen rückgestreuten Strahlung erhalten. Die einzelnen Strahlungsanteile können dabei in allen Stufen zwischen konstruktiv oder destruktiv interferieren, je nach Struktur des Auges innerhalb des kleinsten auflösbaren Volumens. Im Ergebnis erhält man Speckle, die je nach Art der Interferenz (konstruktiv oder destruktiv) heller oder dunkler sein können. Solche Speckles entstehen durch die interferierende Überlagerung der aus dem mit dem verwendeten Meßverfahren aufgelösten Probenvolumen, und sind per se aus dem Gebiet der Ultraschall- und OCT-Messungen bekannt (J. M. Schmitt, "Optical Coherence Tomography (OCT): A Review", IEEE Selected Topics in Quantum Electronics, Vol. 5, Nr. 4, S. 1205-1215, 1999). Ihre minimale Größe ist lateral durch die Fokusgröße und axial durch Kohärenzlänge infolge der genutzten Quellenbandbreite bestimmt. Diese Speckle-Modulationen enthalten zwar Informationen über die Probe, sind eigentlich Teil des Rückstreusignals und auch überwiegend zeitlich stabil, also kein Rauschen im eigentlichen Sinne, stellen jedoch für Grenzflächenbestimmungen und darauf basierender Abstandsbestimmungen ein mindestens ebenso großes Problem dar wie ungenügende Signal/Rausch-Verhältnisse. Deshalb werden Speckle-Modulationen hier als Teil des Rauschens und nicht des Signals betrachtet und ihre Verminderung als eine Verbesserung des Signal/Rausch-Verhältnisses interpretiert.

Bei Rekonstruktion von Signalamplituden sind aufgrund der in den Speckles enthaltenen Phaseninformationen neben dunklen Speckles auch helle Speckles mit unterschiedlichen Vorzeichen der Amplituden möglich. Bei der Streckenmessung am Auge tritt ein Fehler auf, wenn eine für die Streckenmessung zu berücksichtigende Grenzfläche lokal ein dunkles Speckle zeigt, d.h. ein solches mit Amplituden kleiner als der des statistischen Rauschanteile. Diese Grenzfläche wird dann falsch detektiert, und ein Meßfehler ist bei der Streckenmessung die Folge. Die Erfindungsvariante mit einer lateralen Probenverschiebung sorgt nun dafür, daß bei der Bildung des A-Scan-Meßsignals nicht ausschließlich A-Scan-Einzelsignale eingehen, die auf einem dunklen Speckle beruhen; vielmehr sorgt die Zusammenfassung von A-Scan-Einzelsignalen, welche bei unterschiedlichen lateralen Lagen des Fokus gewonnen wurden, automatisch dafür, daß auch helle Speckle zur Bildung des A-Scan-Meßsignals beitragen, so daß der erwähnte Meßfehler vermieden ist. Der durch die laterale Lagevariation verursachte axiale Fehler bei der Grenzflächenbestimmungen an den überwiegend flächigen Augenstrukturen ist dabei deutlich kleiner als derjenige, der aus einer Fehlmessung an einem dunklen Speckle resultieren würde.

Ein Beispiel ist die Grenzfläche der Linse. Der posteriore Linsenradius mit einem typischen Mittelwert von 6 mm ist am Auge eine der am stärksten gekrümmten Strukturen. Ein üblicher lateraler Fokusdurchmesser in ophthalmologischen Geräten ist beispielsweise 25 $\mu$m. Würde nun während der Aufnahme von A-Scan-Einzelsignalen eine laterale Lagevariation beispielsweise über 4 laterale Speckledurchmesser bzw. 100 $\mu$m durchgeführt, so entspräche dies einer axialen Positionsänderung der Grenzfläche um weniger als 1 $\mu$m. Der aus einer potentiellen Fehlmessung an einem dunklen Speckle resultierende Messfehler wäre aber bei Anwendung von OCDR-Verfahren mit üblichen axialen Auflösungen von 10 bis 20 $\mu$m um mindestens eine Größenordnung höher.

Bei Streckenmessungen im Auge ist es erforderlich, daß die die Strecken begrenzenden Grenzflächen mit ausreichendem Signal/Rausch-Verhältnis erfaßt werden. Erzeugt man das A-Scan-Meßsignal aus A-Scan-Einzelsignalen, die sich hinsichtlich der axialen Fokuslage unterscheiden, werden diese A-Scan-Einzelsignale zwar insgesamt dieselbe Probenstruktur wiedergeben, nämlich diejenige Probenstruktur, mit der durch das Interferometer vorgegebene Meßtiefe zugänglich ist, jedoch sind rückstreuende Strukturen, die fokusferner liegen, dann mit geringerer Intensität in einem A-Scan-Einzelsignal vorhanden, als Probenstrukturen, die fokusnäher liegen. Die von der Erfindung in einer weiteren Variante vorgesehene axiale Verschiebung des Fokus während der Aufnahme der Strahlung für die A-Scan-Einzelsignale sorgt somit automatisch dafür, daß für die Erzeugung des A-Scan-Meßsignals ein Satz an A-Scan-Einzelsignalen vorliegt, in denen einzelnen Probenstrukturen unterschiedliche intensive Signale zeigen.

Für beide Varianten ist es nicht nötig, die aktuelle Lage der Fokusverschiebung dem A-Scan-Einzelsignalen zu zuordnen, vielmehr genügt es völlig, die A-Scan-Einzelsignale zum A-Scan-Meßsignal zusammenzufassen, wobei schon eine Addition oder Mittelung, insbesondere von Signalbeträgen. zu einem verbesserten Signal-Rausch-Verhältnis für alle Probenstrukturen innerhalb des erfaßten Meßbereiches führen.

Für die Streckenmessung am Auge sind üblicherweise folgende Strukturen von besonderem Interesse: anteriore und posteriore Fläche der Augenhornhaut, anteriore und posteriore Fläche der Augenlinse und Schichten der Netzhaut, insbesondere die ILM (inner limiting membrane) und das RPE (retinal pigment epithelium). Die Erfindung kann zur Vermessung aus diesen Grenzflächen abgeleiteter Strecken in Kombination mit einem Verfahren bzw. einer Vorrichtung eingesetzt werden, deren Meßtiefe eine unmittelbare Messung der gewünschten Strecken, z. B. der Gesamtlänge des Auges, erlaubt. Für eine solche Anwendung ist die axiale Verschiebung des Fokus während der Messung besonders vorteilhaft. Optional kann die Erfindung aber auch verwirklicht werden mit einer Vorrichtung bzw. einem Verfahren, das einen Teilabschnitt der Augen in einem ersten Meßzustand und einen zweiten Teilabschnitt des Auges in einem zweiten Meßzustand erfaßt. Die laterale bzw. axiale Verschiebung des Fokus findet dann mindestens in einem der Meßzustände statt.

Ein weiterer Aspekt, der bei der Streckenmessung am Auge zu Meßfehlern führen kann, liegt in der Tatsache begründet, daß das Auge zur Vorrichtung bzw. für das Meßverfahren geeignet ausgerichtet werden muß. Man spricht hier vom Justagezustand. Mitunter geht jedoch ein ausreichender Justagezustand noch vor Beginn der eigentlichen Messung aufgrund einer Patientenbewegung wieder verloren, und es muß eine neue Justage durchgeführt werden. Dies stellt natürlich einen Zeitverlust dar. Zudem besteht das Risiko, das der Verlust des ausreichenden Justagezustandes zu spät erkannt und deshalb eine ungültige Messung durchgeführt wird. Hinsichtlich der Linse des Auges ist der Justierzustand dann optimal, wenn die Linse möglichst senkrecht zur einfallenden Meßstrahlung steht, da dann ein starker spekularer Rückreflex entsteht. Bekanntermaßen weichen beim menschlichen Auge die optische Achse durch die Linse und die Sehachse durch das Zentrum schärfsten Sehens, die Fovea centralis, 0 bis 14°, typischerweise um 5° voneinander ab, so daß die Linse gegenüber der Sehachse verkippt ist.. Läßt man nun einen Patienten auf ein Fixierobjekt fixieren, hat dies die Folge, daß auf der Achse der Abbildung des Fixierobjektes einfallende Meßstrahlungen im Allgemeinen auf eine schräg liegenden Linse trifft und der in Richtung der Meßstrahlungsachse detektierbare spekulare (d.h. spiegelartige) Rückreflexanteil gering ist. Ein für die Messung guter Justierzustand ist dann gegeben, wenn die Sehachse durch Verschiebung des Fixierobjektes um den Unterschiedswinkel zwischen Sehachse und optischer Achse gekippt ist, so daß Meßstrahlung in das Auge entlang der optischen Achse der Linse und damit auf eine senkrecht zur Einfallsrichtung liegenden Linse trifft und im Ergebnis einen starken, überwiegend spekularen Rückreflex erzeugt. Möchte man am Auge sowohl die Lage der Linse und die Augenlänge, d. h. den Abstand zwischen Homhautscheitel und Fovea, bestimmen, ist im Stand der Technik entweder eine Folge von zwei Messungen mit dazwischenliegendem Umfixieren des Patienten unerläßlich, oder man muß einen schwächeren Reflex an der Linse akzeptieren. Die Erfindung löst diesen Konflikt nun dadurch, daß mit einer lateralen Verschiebung des Fokus zumindest im Bereich der Linse immer ein starker Rückreflex gesichert ist, da das seitliche Auswandern des Fokus bezüglich der Linse auch Linsenbereiche beleuchtet, welche senkrechter zur Einfallsrichtung der Meßstrahlung stehen, als dies im Bereich der Sehachse der Fall ist. Damit ist sowohl ein Umfixieren des Patienten unnötig, als auch der im Stand der Technik gegebene Auslegungskonflikt behoben. Es ist deshalb eine Weiterbildung der Erfindung besonders bevorzugt, bei der eine Streckenmessung am Auge erfolgt und dabei sowohl die Lage der Netzhaut als auch der Linse erfaßt wird. Wiederum kann dabei eine Vorrichtung oder ein Verfahren zum Einsatz kommen, deren bzw. dessen Meßtiefe in Abstand zwischen Linse und Netzhaut überdeckt, oder es kann eine Umschaltung zwischen Messung der Linsenposition und der Netzhautposition erfolgen. Optional ist natürlich auch ein Zweistrahlverfahren möglich, wobei die laterale Verschiebung des Fokus zumindest an dem Meßstrahl für den Linsenbereich erfolgt.

Im Ergebnis erlaubt die Erfindung eine Reduktion von Fehlern, welche mit dem im Stand der Technik bislang erforderlichen Justierzustand des Auges verbunden waren.

Aufgrund der bekannten doppelbrechenden Wirkung bestimmter Augenstrukturen, wie der Hornhaut oder der Linse oder verschiedener Netzhautschichten, ist eine Veränderung der in den Meßsignalen vorliegenden, störenden Speckle-Modulation auch durch Veränderung des Polarisationszustandes der einfallenden Meßstrahlung möglich. Hinzu kommt, daß die Doppelbrechung auch die Interferenzfähigkeit und damit Detektierbarkeit des rückgestreuten Lichtes stören bzw. mindern kann, so daß die Variationen des Polarisationszustandes der Meßstrahlung höhere Einzelsignale erzielt. Es ist deshalb in der Erfindung zur Verbesserung des Signals ebenfalls vorgesehen, daß während der Aufnahme der A-Scan-Einzelsignale der Polarisationszustand der Meßstrahlung variiert wird. Die obigen Ausführungen hinsichtlich einer nicht nötigen Synchronisierung gelten auch für diese Variante.

Beim Zusammenfassen der A-Scan-Einzelsignale zum A-Scan-Meßsignale wird eine Verbesserung des Gesamtsignals gegenüber den A-Scan-Einzelsignalen erreicht. Das Zusammenfassen kann, wie erwähnt, ganz grundsätzlich im Wege der Addition oder Mittelung erfolgen. Eine nochmalige Verbesserung erhält man, wenn die A-Scan-Einzelsignale selektiert und gewichtet werden. Dazu wird der Signalverlauf der A-Scan-Einzelsignale entsprechend ausgewertet. Beispielsweise ist es möglich, Maximalauswahlen vorzunehmen. Da alle A-Scan-Einzelsignale denselben Meßbereich abdecken, kann man z. B. aus den A-Scan-Einzelsignalen jeweils die maximalen Peaks extrahieren und diese zum A-Scan-Meßsignal zusammenfassen. Auch können Schwellwertauswahlen erfolgen.

Die laterale Verschiebung des Fokus kann auf verschiedene Art und Weise erreicht werden, z. B. durch ein geeignetes den Meßstrahl ablenkendes, angesteuertes Ablenkelement im optischen Aufbau der Vorrichtung. Ohne den Strahlengang des Meßstrahls verschiebende, bewegte Teile kommt man aus, wenn zur lateralen Verschiebung des Fokus ein Fixierbild, welches dem Patienten dargeboten wird, verschoben wird. Der Aufbau ist dann entsprechend einfach, insbesondere wenn das Fixierbild mittels eines von der Steuereinrichtung ansteuerbaren Displays erzeugt wird, das zur Fixierbildverschiebung geeignet angesteuert wird.

Eine weitere, apparativ relativ einfache Variante zur Verschiebung des Fokus liegt darin, ein Optikelement, z. B. eine Linse verstellbar auszugestalten und zur Verschiebung des Fokus zu verstellen. Für eine axiale Verstellung wird z. B. eine Brennweitenveränderung oder axiale Lageveränderung eines refraktiven Elementes (beispielsweise eine Flüssiglinse oder ein Flüssigkristallmodulator) oder einer reflektiven Optik (deformierbarer Spiegel) bewirkt, für eine laterale Verschiebung des Fokus eine Verstellung der Linse quer zu optischen Achse.

Eine weitere Variante kommt ohne zusätzliche Elemente aus: je nach zufälliger, durch unwillküruche Körper- und Augenbewegungen veränderlicher Position und Orientierung des Auges, dominiert nun beispielsweise am Fundus das A-Scan Signal der inneren Grenzmembran gegenüber dem für die Längenmessung relevanten Signal vom retinalen Pigmentepithel (RPE); beispielsweise wie die A-Scan Signale #1 und #7 in der Figur 14 andeuten. Auch zufällige Interferenzen (Speckle) von Licht aus Streuzentren nahe dahinter oder davor können das einzelne resultierende A-Scan KK-Interferogramme auslöschen oder verkleinern. Betrachtet man nun A-Scan Signale dieser Strukturen in der näheren seitlichen Umgebung und/oder zeitlichen Abfolge, so kann man von solchen Zufälligkeiten unabhängig werden und man findet die richtige Zuordnung. Insbesondere kann eine Summierung der räumlich und/oder zeitlich eng benachbarten Signale solche Zufälligkeiten ausmitteln. Hierbei müssen die räumlich transversal versetzten A-Scan Signale keinesfalls schon ein die transversalen anatomischen Strukturen erkennen lassendes Bild ergeben; vielmehr genügt es, so viele A-Scans vom Fundus aufzunehmen, bis man im Bereich des Fundus beispielsweise 2 um etwa die Retinadicke von rund 0,3mm distanzierte Messsignale sieht. Man kann alternativ die einzelnen A-Scan Signale summieren und sich an der Stärke der Summensignale orientieren. Beispielsweise ist am Fundus das A-Scan Signal von dem retinalen Pigmentepithel meist das stärkste Signal und dominiert daher auch im Summensignal. Das zeigt auch Figur 14: Summiert man die jeweils zu IG und RG gehörigen Signale, dominiert die Summe von RP gegenüber der von IG.

Für Ausführungsformen, in denen der interferometrisch zugängliche Meßbereich nicht alle Grenzflächen, welche bei der Streckenmessung relevant sind, gleichzeitig erfassen kann, ist es zu bevorzugen, den Fokus zusätzlich zur Lageverstellung während der Aufnahme der rückgestreuten Strahlung auch noch in verschiedene axial beabstandete Teilbereiche des Objektes zu verstellen, und die Verschiebung des Fokus während der Aufnahme der Strahlung in zumindest einem der Teilbereiche auszuführen. Dies ist ein Beispiel für die zuvor erwähnten Meßzustände.

Bei einer lateralen Verschiebung des Fokus ist es vorteilhaft, diese mit einer Verschiebegeschwindigkeit auszuführen, die kleiner als der Quotient aus dem halben Fokusdurchmesser und einer Aufnahmedauer der Strahlung für ein A-Scan-Einzelsignal ist, und diese Verschiebegeschwindigkeit hat sich besonders vorteilhaft zur Reduktion der geschilderten Speckle-induzierten Fehler erwiesen. Besonders bevorzugt ist, daß der Quotient kleiner ist als 10% des Verhältnisses aus Fokusdurchmesser und Aufnahmedauer der Strahlung für ein A-Scan-Einzelsignal.

Für die einzelnen A-Scan-Einzelsignale ist es in den Ausführungsformen der Erfindung nicht erforderlich bzw. gar nicht bekannt, wie die aktuelle Verschiebung des Fokus war. Dennoch ermöglicht die Erfindung es, weitere Informationen über die Augenlinse zu gewinnen, indem für alle Einzelsignale die Positionen der Linsenvorderfläche und -rückfläche ermittelt werden. Für jedes Einzelsignal liegt somit ein Paar aus Positionen der Linsenvorderfläche und Positionen der Linsenrückfläche vor. Die Differenz zwischen der am meisten anterior liegenden der ermittelten Position der Linsenvorderfläche und der am meisten posterior liegenden der ermittelten Position der Linsenrückfläche gibt die Dicke der Augenlinse wieder.

Trägt man die Paare aus ermittelter Position der Linsenvorderfläche und ermittelter Position der Linsenrückfläche in einem Diagramm auf, wobei entlang einer Diagrammachse die Position der Linsenvorderfläche und entlang der anderen Diagrammachse die Position der Linsenrückfläche aufgetragen ist, erlaubt eine Verbindung oder Interpolation der erhaltenden Punkte zu einer Kurve es, ein Maß für die Verkippung der Linse gegenüber der Sehachse zu bestimmen, indem der maximale Abstand der Kurvenpunkte von einer Symmetrieachse der Kurve ausgewertet wird.

Die beiden obengenannten Weiterbildungen sind Beispiele dafür, daß auch ohne Zuordnung der aktuellen Fokusverschiebung bzw. des aktuellen Polarisationszustandes aus den A-Scan-Einzelsignalen weitergehende Informationen über das Auge gewonnen werden können.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind.

Nachfolgend wird die Erfindung beispielsweise anhand der beigefügten Zeichnungen, die auch erfindungswesentliche Merkmale offenbaren, noch näher erläutert. Es zeigen:

Fig. 1 eine Schemazeichnung einer Vorrichtung zur Messung von Strecken am Auge,
Fig. 2 und 3 detailliertere Darstellungen der Vorrichtung der Fig. 1,
Fig. 4a und 4b Signale, die beim Betrieb der Vorrichtung der Fig. 1, Fig. 2 oder Fig. 3 erhalten werden,
Fig. 5 und 6 eine Abwandlung der Vorrichtung der Fig. 2 und 3,
Fig. 7 eine detailliertere Darstellung der Vorrichtung der Fig. 5 und 6,
Fig. 8 eine weitere Modifikation der Vorrichtung der Fig. 1,
Fig. 9 eine detailliertere Darstellung einer weiteren Bauweise der Vorrichtung der Fig. 1,
Fig. 10 Fixiermuster, die beim Betrieb einer der Vorrichtungen verwendet werden können,
Fig. 11 eine weitere Variante der Vorrichtung der Fig. 1,
Fig. 12 eine detailliertere Darstellung der Vorrichtung der Fig. 11,
Fig. 13 ein Schema zur Auswertung der A-Scan-Signale,
Fig. 14 eine Darstellung verschiedener A-Scan-Signale,
Fig. 15 eine weitere Ausführung der Erfindung mit einer doppelbrechenden Optik,
Fig. 16 eine weitere Ausführung der Erfindung mit einer diffraktiven Optik,
Fig. 17 eine Abwandlung der Ausführung aus Fig. 15 und
Fig. 18 eine weitere Ausführung der Erfindung mit zwei FD-Kurzkohäreninzinterferometem.

Bei der interferometrischen Messung axialer Streuproflle, wie sie bei der optischen Kohärenzdomänenreflektrometrie auftritt, ist die axiale Auflösung, d. h. die Auflösung in Tiefenrichtung, im Wesentlichen durch das Kohärenzverhalten der Quelle gegeben. Die Auflösung ist umgekehrt proportional zur Gesamtbreite des zur Vermessung des Streuprofils verwendeten Spektrums. Quer dazu, d. h. in lateraler Richtung ist die erreichbare Auflösung durch die laterale Ausdehnung des Fokus gegeben, die hier als Fokusdurchmesser bezeichnet wird. Tatsächlich liegt natürlich eine Strahltaille vor und der Fokusdurchmesser wird üblicherweise als diejenige Größe der Strahltaille aufgefaßt, bei der die Strahlungsintensität auf einen bestimmten Wert, z. B. $1/e^2$, abgefallen ist.

Die von einer Stelle eines Objektes rückgestreute Strahlung ist eine Überlagerung der aus dem aufgelösten Volumen rückgestreuten Strahlungsanteile. Diese Strahlungsanteile können in allen Stufen zwischen konstruktiv und destruktiv interferieren, wodurch sich bekannte Speckle bilden. Bei einer konstruktiven Interfrenz liegen helle Speckle vor, bei einer destruktiven Interferenz dunkle Speckle. Ob eine konstruktive oder destruktive Interferenz auftritt, hängt von der Objektstruktur innerhalb des aufgelösten Volumens ab. Weiter ist die Intensität der rückgestreuten Strahlung davon abhängig, wie nahe eine Objektstruktur dem Fokus der Meßstrahlung liegt. Fokusfemere Objektstrukturen führen zu geringeren Rückstreuintensitäten und damit zu geringer starken Interferenzsignalen. Eine weitere Variation der Interferenzsignalstärke kann durch Doppelbrechungseffekte in der Probe entstehen.

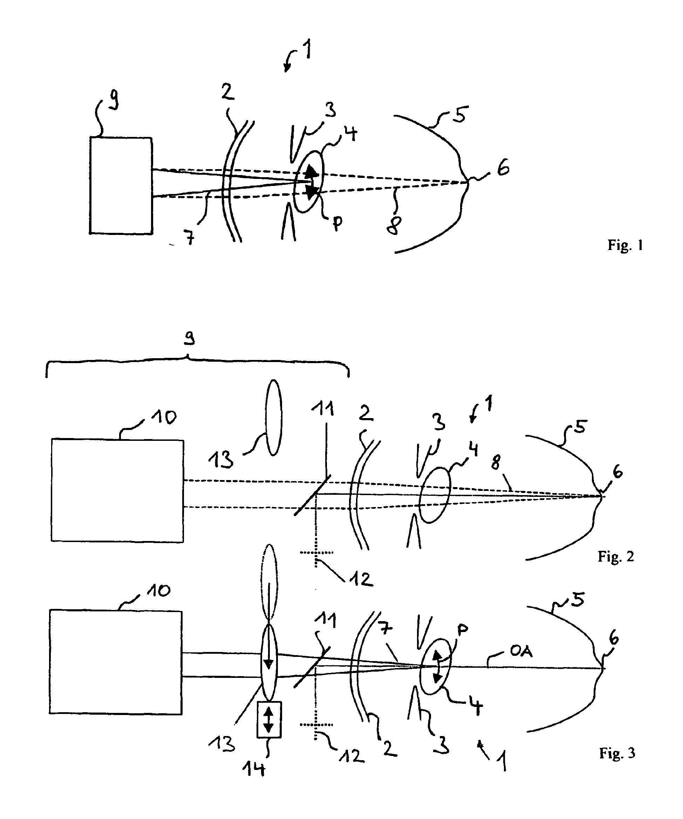

Um nun bei der interferometrischen Objektvermessung das Signal einer zu vermessenden Grenzfläche zu maximieren, wird gemäß Fig. 1 während der Messung eines axialen Streuprofils der Fokus verschoben oder der Polarisationszustand des Meßstrahls geändert. In Analogie zur Ultraschallmeßtechnik werden solche axiale Streuprofile auch als A-Scans bezeichnet, weshalb hier von einem A-Scan-Meßsignal die Rede ist. Eine Minderung des Interferenzsignals und damit des Meßsignals durch ein zufälliges dunkles Speckle an der Objektstruktur wird dadurch vermieden, daß der Fokus während der Messung lateral verschoben wird. Eine Maximierung des Signals hinsichtlich des Abstandes zum Fokus wird dadurch erreicht, daß der Fokus während der Messung axial verschoben wird. Negativ die Signalintensität beeinflussende Doppelbrechungseffekte werden dadurch vermieden, daß der Polarisationszustand des Meßstrahls während der Messung geändert wird.

Fig. 1 zeigt einen Schnitt durch ein Auge 1, von dem exemplarisch und schematisch Hornhaut 2, Iris 3, Linse 4, Netzhaut 5 und Fovea 6 eingezeichnet sind. In dem in Fig. 1 dargestellten Zustand wird das Auge mit zwei Meßstrahlen 7 und 8 vermessen. Der Meßstrahl 7 ist in die Linse 4 fokussiert, der Meßstrahl 8 an der Fovea 6. Die Meßstrahlen 7 und 8 stammen aus einer Meßvorrichtung 9, das für die optische Kohärenzdomänenreflektrometrie (OCDR) ausgebildet ist und damit eine Tiefenauslösung der erfaßten Bereiche, in diesem Falle der Linse 4 und der Netzhaut 5 bewirkt. Hinsichtlich dabei möglicher OCDR-Prinzipien und Realisierungen sei auf folgende Literatur verwiesen: WO2007065670 oder Fercher et al., "Measurement of Intraocular Distances by Backscattering Spectral Interferometry", Opt. Comm. 117,43.

Die Meßvorrichtung 9 weist ein (in Fig. 1 nicht weiter dargestelltes) Interferometer auf und nimmt Anteile der Meßstrahlen 7 und 8 auf, die aus dem jeweiligen Fokusvolumen rückgestreut wurden. Um zu verhindern, daß die Rückstreuung zufälligerweise als dunkles Speckle ausgebildet ist, wird der Meßstrahl 7 während der Messung des Streuprofils, d. h. während der Erfassung des A-Scan-Meßsignals, lateral verschoben, so daß das Fokusvolumen, z. B. in der Linse 4, wandert. Dies ist in Fig. 1 durch einen Pfeil P symbolisiert. Für die Gewinnung des A-Scan-Meßsignals werden eine Vielzahl an Streuprofilen erfaßt, die A-Scan-Einzelsignale darstellen. Diese werden auch noch zu beschreibende Art und Weise zum A-Scan-Meßsignal zusammengefaßt.

Anders als bei einem Scannen, welches zur Bildgewinnung verwendet wird, wie sie beispielsweise bei der optischen Kohärenztombographie (OCT) eingesetzt wird, ist die Lateralverschiebung des Fokus nicht zur Aufnahme der Strahlung, d. h. der A-Scan-Meßsignale synchronisiert. Diese nicht vorhandene Synchronisation drückt sich dadurch aus, daß bei der Zusammensetzung der A-Scan-Einzelsignale die Lateralverschiebung des Fokus nicht berücksichtigt wird. Insbesondere kann die Lageverschiebung des Fokus mehrfach bzw. zyklisch um eine mittlere Lage erfolgen, ohne daß die Zyklen mit der Aufnahme der A-Scan-Einzelsignale synchronisiert sind.

Die Verschiebung erfolgt mit einer Geschwindigkeit, die so groß ist, daß während der Dauer, die für die Aufnahme aller A-Scan-Einzelsignale eines A-Scan-Meßsignals nötig ist, der Fokus im Objekt um mindestens den halben Fokusdurchmesser (vorzugsweise höchstens 125 Fokusdurchmesser) wandert, wobei jedoch die Verschiebung pro Dauer der Strahlungserfassung der A-Scan-Einzelsignal vorzugsweise den oben genannten Begrenzungen unterliegt. Durch dieses Wandern des Fokusdurchmessers ist sichergestellt, daß nicht alle A-Scan-Einzelsignale von demselben Speckle stammen. Ab einer Verschiebung von einem Fokusdurchmesser ist es mit gesteigerter Sicherheit vermieden, daß ausschließlich ein destruktiv interferierendes Objektvolumen zum Meßsignal einer Objektstruktur beiträgt, also an einer bestimmten axialen Position nicht irrtümlich aufgrund destruktiver Interferenz das NichtVorhandensein einer Objektstruktur angenommen wird. Für die Verschiebung kann in einer bevorzugten Variante auch eine Maximalauslenkung definiert werden. Für sie gilt dann:

$$\text{Maximalauslenkung [in lateralen Speckledurchmessern]} =$$
$$\text{sqrt} ( R^2 - ( R^2 - \text{Axialauflösung} )^2 ) / \text{Lateralauflösung}$$

Für das bereits angegebene Beispiel (posteriorer Linsenradius R = 6 mm, Lateralauflösung 20 $\mu$m, Axialauflösung 20 $\mu$m) erhält man also 24 laterale Fokusdurchmesser als Maximalauslenkung vom Linsenapex aus erhalten. Wird die Maximalauslenkung größer entspricht oder übersteigt der axiale Positionsbestimmungsfehler aufgrund der Lateralauslenkung demjenigen durch Fehlmessung an einem dunklen Speckle. Nimmt man die für Biometrieanwendungen sinnvollen Grenzen von 6 und 100 $\mu$m Axialauflösung und 10 bis 20 $\mu$m Lateralauflösung, so erhält man Maximalauslenkungen von 13 bis 110 Fokusdurchmessem vom Linsenapex aus.

Will man eine nachfolgend noch erläuterte, statistische Auswertung vornehmen, um Information über die Linsengeometrie ableiten, muß die Maximalauslösung deutlich über den genannten Werten liegen. Es ist also zweckmäßig in einem ersten Betriebsmodus für Längenmessungen die genannten Maximalauslenkungen einzuhalten und in einem zweiten Betriebsmodus für die Ermittlung von Linsengeometrie größere Auslenkungen vorzunehmen. Die Steuereinrichtung schaltet dann zwischen den beiden Betriebsmodi um.

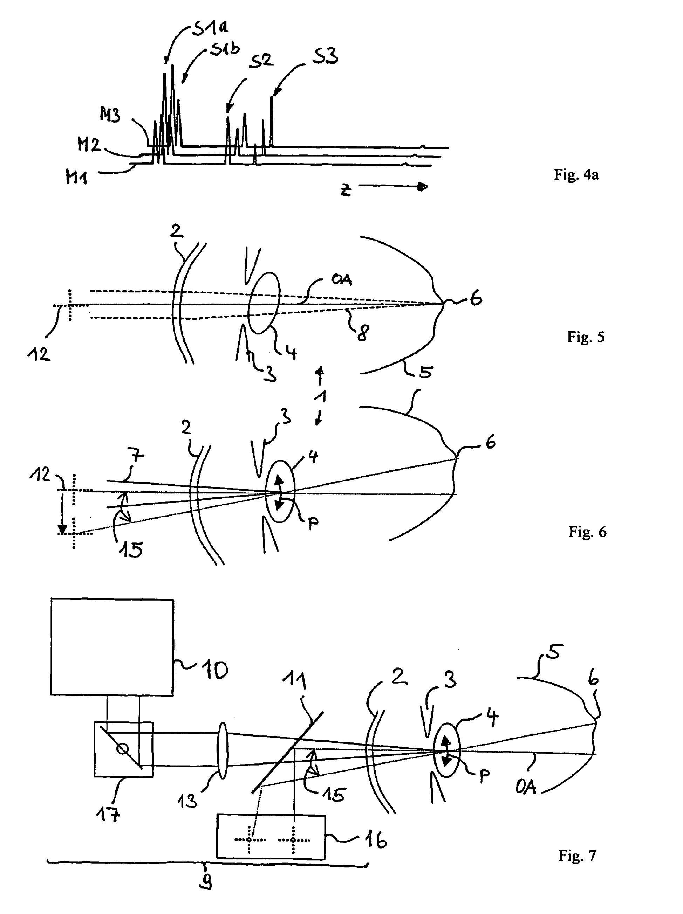

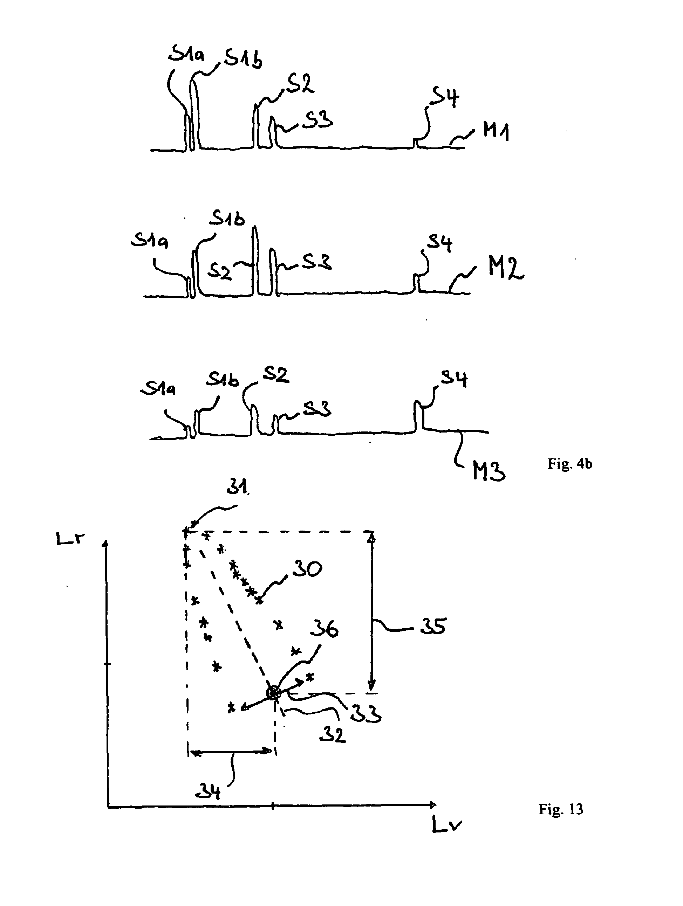

Diese Wirkung ist in Fig. 4b dargestellt, die drei A-Scan-Einzelsignale M1, M2 und M3 in perspektivisch gestaffetter Darstellung zeigt. Die Meßsignale M1, M2 und M3 sind längs der Tiefe, d. h. längs der z-Koordinate des Auges 1 aufgetragen. Wie zu sehen ist, haben die Einzelsignale M1, M2 und M3 jeweils mehrere Spitzen. Exemplarisch sind vier Spitzen S1a, S1b, S2 und S3 eingezeichnet. Die Spitzen S1a und S1b sind durch Reflexe an der Vorder- bzw. Rückseite der Hornhaut 2 bedingt. Die Spitze S2 rührt von einem Reflex an der Vorderseite der Linse 4, die Spitze S3 von einem Reflex an der Rückseite her. Wie zu sehen ist, zeigen alle drei perspektivisch gestaffelten Einzelsignale M1, M2 und M3 die Spitzen S1a, S1b, S2 und S3 jeweils an derselben z-Koordinate, jedoch mit unterschiedlicher Intensität. Dieser Intensitätsunterschied rührt daher, daß durch die laterale Verstellung der Fokusposition (symbolisiert durch den Pfeil P in Fig. 1) Speckle unterschiedlicher Helligkeit bei der Erzeugung des jeweiligen Einzelsignals M1, M2 und M3 erfaßt wurden, so daß die Streuintensitäten variieren. Beispielsweise stammt die Spitze S3 des Einzelsignals M1 von einem deutlich dunkleren Speckle her, als die Spitze S3 des Einzelsignals M3. Spitzen aufgrund von Volumenstreuung, beispielsweise in der Linse zwischen S2 und S3, sind der Übersichtlichkeit in den Darstellungen (Fig. 4a und 4b) weggelassen, jedoch gelten die am Beispiel der Speckles an den Grenzflächen diskutierten signalverbessemden Aspekte natürlich auch für diejenigen aus streuenden Volumina.

Fügt man die Einzelsignale M1, M2 und M3 zum Meßsignal zusammen, hat dieses ein deutlich verbessertes Signal/Rausch-Verhältnis gegenüber einer Messung mit festem Fokus. Das Zusammenfügen kann dabei auf verschieden Art und Weise erfolgen, beispielsweise durch eine Mittelung. Auch können Maximumauswahlen vorgenommen werden, beispielsweise kann die Spitze S1a aus dem Einzelsignal M2, die Spitze S1b aus dem Einzelsignal M3, die Spitze S2 aus dem Einzelsignal M1 sowie die Spitze S3 aus dem Einzelsignal M3 ausgewählt werden. Für solche Auswahlen sind dem Fachmann verschiedenste Ansätze bekannt, mit denen das Signal/Rausch-Verhältnis nochmals gegenüber einer Mittelung gesteigert werden kann.

Fig. 1 zeigt, daß die Meßvorrichtung 9 zwei verschiedene Teilbereiche des Auges 1 erfaßt, nämlich die Linse 4 und die Netzhaut 5 (letzteres im Bereich der Fovea 6) und dazu zwei getrennte Meßstrahlen 7 und 8 einsetzt. Dies geschieht für Ausführungsformen, bei denen der Meßbereich, der in axialer Richtung überdeckt werden kann, nicht dazu ausreicht, zwei interessierende Teilbereiche des Auges 1 gleichzeitig oder das ganze Auge zu erfassen. Die laterale Verschiebung gemäß dem Pfeil P ist in Fig. 1 lediglich exemplarisch für einen der Teilbereiche eingetragen. Natürlich kann auch der Meßstrahl 8 entsprechend lateral bewegt werden.

Bei einer Meßvorrichtung 9, deren axiale Meßtiefe genügt, um ein Objekt im gewünschten Bereich vollständig zu erfassen, wird hingegen mit einem Meßstrahl gearbeitet. Dann wird der Meßbereich geeignet durchgestimmt. Bei einem TD-OCDR wird dazu die Referenzarmlänge geeignet so verstellt, daß sie z. B. die gesamte Augenlänge abdeckt. Bei einem SD-OCDR-System ist analog die Bandbreite der spektralen Aufgliederung bzw. die spektrale Auflösung entsprechend zu wählen, bei einer SS-OCDR-System die Bandbreite der spektralen Durchstimmung der Quelle. Natürlich kann der zweite Meßstrahl auch entfallen, wenn ein Auge nicht vollständig erfaßt werden soll, sondern z. B. nur ein Teilabschnitt. Dies ist am Auge 1 der Fall, wenn z. B. die Netzhaut 5 oder die Linse 4 nicht erfaßt werden soll. Um in diesem Fall die Signalintensitäten zu optimieren, wird der Fokus axial im Auge verstellt.

Die Wirkung dieser Verstellung ist in Fig. 4b gezeigt, welche wiederum drei A-Scan-Einzelsignale M1, M2 und M3 zeigt, die längs der Tiefe, d. h. längs der z-Koordinate des Auges 1 aufgetragen sind. Die Bezeichnung der Spitzen (Peaks) der Einzelsignale M1, M2 und M3 ist analog zur Fig. 4a. Wie zu sehen ist, tritt zusätzlich zu den Spitzen S1a, S1b, S2 und S3 noch eine Spitze S4 hinzu, die von der Rückstreuung an der Augennetzhaut verursacht ist. Während Aufnahme der Einzelsignale M1, M2 und M3 wird der Fokus in Richtung Netzhaut verschoben, wodurch die Intensität der Spitzen sich verändert. Bei stark anteriorer Lage des Fokus, wie sie für das Einzelsignal M1 gegeben ist, ist die Spitze S4 nur äußerst schwach ausgeprägt, die Spitzen S1a und S1b hingegen sehr groß. Eine stark posteriore Lage des Fokus betont hingegen die Spitze S4.

Fügt man nun wiederum die Einzelsignale M1, M2 und M3 zum Meßsignal zusammen, hat dieses wiederum ein deutlich verbessertes Signal/Rausch-Verhältnis. Obige Ausführungen hinsichtlich der Fig. 4a gelten natürlich analog für die Fig. 4b. Darüber hinaus sei angemerkt, daß in beiden Figuren die Zahl von drei Einzelsignalen rein zur Veranschaulichung gewählt wurde. Tatsächlich können natürlich beliebige Zahlen an Einzelsignalen verwendet werden, und üblicherweise liegt die Zahl deutlich über drei, z. B. bei einigen Hundert oder Tausend.

Die Fig. 2 und 3 zeigen exemplarisch eine Realisierung der Meßvorrichtung 9 für das Konzept gemäß Fig. 1 in zwei unterschiedlichen Betriebszuständen. In einem ersten Zustand, der in Fig. 2 dargestellt ist, wird der Meßstrahl 8 von der Meßvorrichtung 9 abgegeben, die exemplarisch ein OCDR-Interferometer 10 aufweist. Um das Auge 1 des Patienten so auszurichten, daß der Meßstrahl 8 auf die Fovea 6 fällt, ist über einen Strahlteiler 11 ein Fixierbild 12 eingespiegelt, auf das der Patient fixiert. Dadurch richtet er zum einen das Auge 1 so aus, daß der Meßstrahl 8 auf die Fovea 6 fällt. Zugleich bewirkt die Fixierung auf das Fixierbild 12, daß die Linse 4 in einem Zustand ist, welcher die Lage des Fokus des Meßstrahls 8 exakt in der Fovea 6 sicherstellt.

Die Meßvorrichtung 9 weist weiter eine einschwenkbare Linse oder Optik 13 auf, deren Einschwenken bewirkt, daß der Fokus des Meßstrahls in der Linse 4 liegt. Der Meßstrahl wird somit im Zustand gemäß Fig. 3 zum Meßstrahl 7. Die Vorrichtung realisiert also die in Fig. 1 eingezeichneten Meßstrahlen 7 und 8 sequentiell, d. h. nicht gleichzeitig. Natürlich kann, wie später noch ausgeführt wird, auch eine gleichzeitige Abgabe beider Meßstrahlen 7 und 8 erfolgen.

Die Meßvorrichtung 9 weist weiter einen Stellantrieb 14 für die Linse 13 auf, der im eingeschobenen Zustand die Lage der Linse quer zur optischen Achse OA der Meßvorrichtung 9 verstellt. Dadurch wandert die Lage des Fokus lateral in der Linse 4 gemäß dem Pfeil P. Die Verschiebung der Linse 13 senkrecht zur optischen Achse ist natürlich nur eine von vielen Möglichkeiten, den Fokus lateral wandern zu lassen. Auch andere optische Elemente können dazu eingesetzt werden, z. B. Ablenkspiegel, eine oder mehrere planparallele Platten, welche unterschiedlich verkippt werden etc.

Eine Abwandlung der Bauweise der Fig. 3 optimiert die Intensität der Meßstrahlung, die von einer Objektstelle im Auge 1 zurückgestreut wird, durch Variation des Polarisationszustandes des Meßstrahls 7. In dieser Bauweise sind die Linse 13 sowie der Stellantrieb 14 durch eine Einrichtung zur Änderung des Polarisationszustandes des Meßstrahls ersetzt. Der Polarisationszustand wird analog zur Fokuslage während der Messung variiert, so daß im Ergebnis das A-Scan-Meßsignal aus A-Scan-Einzelsignalen zusammengesetzt wird, welche bei verschiedenen Polarisationszuständen des Meßstrahls gewonnen wurden. Für diese Ausführungsform ersetzt also die Variation des Polarisationszustandes die Lageveränderung des Fokus. Ansonsten gelten die hier gemachten Ausführungen voll umfänglich auch für diese Variante.

Einen Ansatz, bei dem keine optischen Bauteile der Meßvorrichtung 9 bewegt werden müssen, zeigen die Figuren 5 und 6. Fig. 5 zeigt schematisch einen Zustand ähnlich der Fig. 2, wobei Elemente wie der Strahlteiler 11 und andere Bauteile der Meßvorrichtung 9 der Übersichtlichkeit halber weggelassen wurden. Die Lateralverschiebung des Fokus wird nun nicht durch Bewegung eines den Meßstrahl 7 bzw. 8 in das Auge 1 abbildenden Elementes der Meßvorrichtung 9 bewirkt, sondern durch Verschiebung des Fixierbildes 12. Dies hat zur Folge, daß der Patient während der Meßsignalaufnahme umfixiert und dadurch im Ergebnis der Fokus des Meßstrahls 7 in der Linse 4 lateral wandert.

Der Patient kippt sein Auge und damit seine Sehachse um einen Winkel 15, was eine Verschiebung der Lage des Fokus des Meßstrahls 7 in der Linse 4 zur Folge hat.

Die laterale Positionsänderung des Fokus während der Messung bewirkt auch eine Variation in der Ausrichtung des Meßstrahls zu eventuell gekrümmten Grenzflächen im Auge, z. B. der Linse 4. Damit wird es möglich, starke Rückstreuungen mit überwiegend spekularen Anteilen zu erreichen, die beispielsweise mit einer groben Vorjustage nicht erzielt wurden oder durch eine zwischenzeitliche Relativbewegung von Meßvorrichtung und Objekt, z. B. einer Augenbewegung, wieder verloren gingen. Diese Signalverbesserungen durch die Lageänderung des Fokus des Meßstrahls während der Messung werden durch eine Umfixierung, d. h. eine Verschiebung des Fixierbildes 12, besonders unterstützt, insbesondere bei der Streckenmessung, bei den Grenzflächen der Linse 4 relevant sind, da die optische Achse der Linse 4 meist gegenüber der Sehachse des Auges verkippt ist.

Fig. 7 zeigt ein Ausführungsbeispiel für die Meßvorrichtung 9 zur Realisierung der Lageverschiebung gemäß den zuvor geschilderten Prinzipien. Elemente der Meßvorrichtung 9 der Fig. 7, die denen der Fig. 2 und 3 entsprechen, sind mit denselben Bezugszeichen versehen und werden zur Vermeidung von Wiederholungen nicht noch einmal beschrieben. Um den Fokus verschieben zu können, ist zur Erzeugung des Fixierbildes 12 eine Fixierbildeinrichtung 16 vorgesehen, die ein Fixiermuster, z. B. das in Fig. 7 dargestellte Kreuz, an unterschiedlichen Stellen erzeugen kann, z. B. durch ein entsprechendes Anzeigeelement, auf dem das Fixiermuster verschieblich ist. Auf eine Transversalverschiebung der Linse 13 und den entsprechenden Antrieb 14 kann dann verzichtet werden. Dies soll allerdings nicht heißen, daß die Linse 13 nicht aus dem Strahlengang geschwenkt werden kann, wie in Fig. 2, um den Meßstrahl 8 zu erzeugen.

Weiter ist in der Bauweise der Fig. 7 noch ein Ablenkspiegel 17 vorhanden, der für die Fokuslateralverschiebung zusätzlich zur durch die Umfixierung erzielten Fokuslageverschiebung genutzt werden kann, insbesondere zur Reduzierung der Speckle-Modulation des Signals.

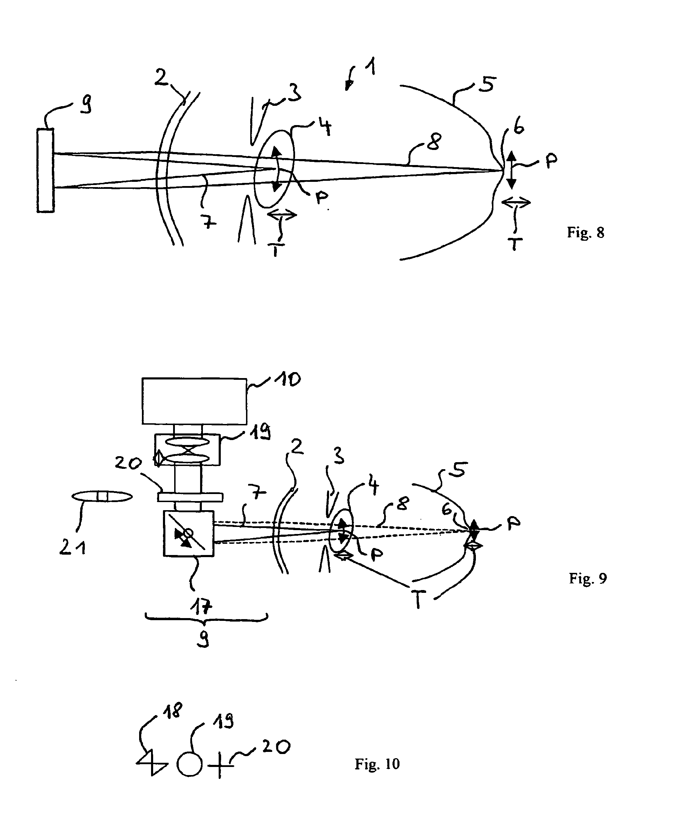

Die Fig. 8 und 9 betreffen Ausführungsformen, bei denen zwei Teilbereiche des Objektes, also im Ausführungsbeispiel des Auges 1 simultan erfaßt werden. Die Meßstrahlen 7 und 8 werden gleichzeitig abgegeben und in die Linse 4 bzw. auf die Netzhaut 5 fokussiert. Während der Strahlungsaufnahme für die A-Scan-Meßsignale für die beiden Teilbereiche, d. h. eines Meßsignals für jeden Teilbereich, wird der Fokus wie erwähnt verschoben. Die Fokusverschiebung erfolgt dabei zum einen lateral, indem der in der Bauweise der Fig. 7 noch feststehende Umlenkspiegel nun schwenkbar ausgestaltet ist. Seine Bewegung sorgt für die Lateralverschiebung des Fokus sowohl des Meßstrahls 7 als auch des Meßstrahl 8. Dies ist durch die beiden Pfeile P in Fig. 8 dargestellt.

Zusätzlich wird in der Bauweise der Fig. 9 noch eine axiale Verschiebung des Fokus vorgenommen. Dazu ist eine verstellbare Optik 19 mit einer verschiebbaren Linse vorgesehen, welche die Foki der Meßstrahlen 7 und 8 simultan längs der optischen Achse verstellt. Dies ist durch einen axialen Meßbereich T in Fig. 9 symbolisiert. Anders als anhand Fig. 4b erläutert, erfolgt die axiale Fokusverstellung also nicht über einen großen Bereich, um die Rückreflexe von unterschiedlichen Grenzflächen, welche axial beabstandet sind, zu optimieren, sondern in der Bauweise der Fig. 9 in einem gegenüber der Meßtiefe kleinen Bereich. Diese Verschiebung hat, wie die laterale Verschiebung auch, den Vorteil, daß vermieden wird, daß ein dunkles Speckle zu einer unerwünschten Meßsignalminderung führt. Die Bauweise der Fig. 9 ist also ein Beispiel dafür, daß die laterale Verschiebung des Fokus und die axiale Verschiebung des Fokus auch kombiniert werden können. Weiter ist die Bauweise der Fig. 9 ein Beispiel dafür, daß die axiale Verschiebung des Fokus (natürlich auch ohne laterale Verschiebung des Fokus) grundsätzlich auch in einem Bereich erfolgen kann, der klein gegen den Abstand der zu ermittelnden Strecken ist.

Grundsätzlich können in der Bauweise der Fig. 9, wie in den anderen Ausführungsformen auch, TD-, SS- oder SD-Ansätze zur Anwendung kommen. Entsprechende Aufbauten für die Meßvorrichtung 9 sind dem Fachmann bekannt.

Fig. 9 zeigt exemplarisch weitere Details der Meßvorrichtung 9, die jedoch auch anderweitig zum Einsatz kommen können. Um die Meßstrahlen 7 und 8 gleichzeitig zu erzeugen, weist die Meßvorrichtung 9 nach dem Interferometer 10 ein entsprechend aufteilendes Element vor, z. B. ein diffraktives optisches Element (DOE) 20 oder eine exemplarisch links neben dem DOE eingezeichnete segmentierte Linse 21. Das aufteilende Element erzeugt den unterschiedlich fokussierten doppelten Meßstrahl 7, 8.

Die in Fig. 10 dargestellten Muster 18, 19 und 20 sind Fixierungsmuster, die bei einem zweiachsig verstellbaren Umlenkspiegel 17 erzeugt werden können, um als Fixierbild zu dienen. Hierzu wird ein Meßstrahl, den ein Patient zumindest mit geringer Empfindlichkeit noch sehen kann, beispielweise im Spektralbereich 700..850 nm, verwendet, oder bei Verwendung unsichtbarer Meßstrahlung (beispielsweise 1060 nm) erfolgt eine geeignete Überlagerung mit einem sichtbaren Fixationsstrahl (beispielsweise 635 nm) vor der Ablenkeinrichtung 17, beispielsweise mittels eines dichrioitischen Strahlteilers (nicht dargestellt).

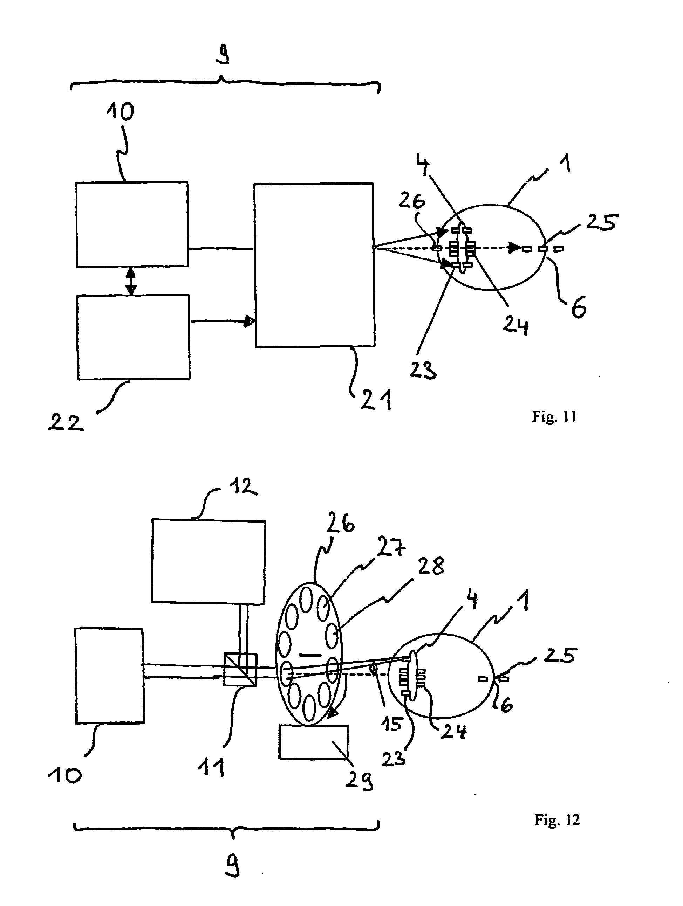

Die Verschiebung des Fokus, welche natürlich eine Verstellung der Fokuslage darstellt, kann gemäß der Ausführungsform der Fig. 11 auch durch eine Programmoptik 21 erfolgen, welche hinsichtlich lateraler Fokusposition, axialer Fokusposition, Fokusgröße, Polarisationszustand oder Anzahl der Foki bestimmte fest eingestellte, also programmierte Änderungen ausführt. Nachfolgend wird als Änderung eine Verschiebung des Fokus, sowohl lateral als auch axial, erläutert. Dies ist jedoch rein exemplarisch zu verstehen. Die Programmoptik durchläuft ein Programm der Fokusverstellungen. Sie wird dabei von einer Steuereinrichtung 22 entsprechend aktiviert. Der in Fig. 10 für die Steuereinrichtung 22 des Meßgerätes 9 zwischen Steuereinrichtung 22 und Programmoptik 21 gezeichnete Pfeil soll symbolisieren, daß die Steuereinheit 22 die Programmoptik 21 z. B. lediglich ein- bzw. ausschaltet, auf jeden Fall keine Informationen über den aktuellen Programmzustand der Programmoptik vorgibt oder ausliest - dies deshalb, da die Steuereinrichtung 22 die aktuelle Fokusverschiebung (oder Polarisationsvariation) bei der Erzeugung der A-Scan-Meßsignale bzw. der A-Scan-Einzelsignale überhaupt nicht verwertet.

Die Programmoptik 21 verstellt die Lage des Fokus im Auge 1 an die Stellen, die in Fig. 11 durch kleine Kästchen symbolisiert sind. Der Fokus liegt also an verschiedenen Stellen an der Vorderfläche 23 der Linse 4, an verschiedene Stellen an der Rückfläche 24 der Linse, an eine Stelle im Bereich des Hornhautscheitels 26 sowie an mehreren Stellen im Bereich der Netzhaut 25. Die Dauer eines jeden Programmschrittes ist vorzugsweise zeitlich lang genug, um mindestens ein A-Scan-Einzelsignal aufnehmen zu können, wobei allerdings diese Aufnahme nicht synchron zu den Programmschritten erfolgt. Es können also durchaus A-Scan-Einzelsignale vorliegen, während deren Strahlungsaufnahme die Programmoptik von einem Programmschritt zum nächsten wechselt.

Es sei hier noch einmal betont, daß die Verstellung der Lage des Fokus nicht primär dazu dient, einen größeren Meßbereich, der zur Messung von Strecken oder Teilstrecken im Auge erforderlich ist, abzudecken. Der Meßbereich wird vielmehr durch entsprechende Ausgestaltung des Interferometers 13 abgedeckt. Je nach Ansatz (TD-, SD- oder SS-OCDR) wird also das Interferometer durchgestimmt, die spektrale Zusammensetzung der Meßstrahlung variiert oder ein Spektrum der Interferenzstrahlung aufgenommen. Die Verstellung der Fokuslage dient auch nicht dazu, Bildinformation durch Abrastem quer zur Haupteinfallsrichtung der Strahlung zu erzeugen, sondern soll vielmehr A-Scan-Einzelsignale bereitstellen, die jeweils unterschiedliche Bereiche des Auges mit unterschiedlicher Empfindlichkeit abfühlen und somit nach Zusammenfassung der A-Scan-Einzelsignale zu einem A-Scan-Meßsignal führen, das ein deutlich verbessertes Signal/Rausch-Verhältnis hat. Allerdings können auch Abweichungen zwischen den Einzel-A-Scans statistisch ausgewertet werden, beispielsweise um Form- und Lageinformationen zu gewinnen, ohne auf Synchronisation und Bildgebung zurückzufallen.

Fig. 12 zeigt exemplarisch eine mögliche Ausführung der Programmoptik. Sie ist hier als Linsenrad 26 realisiert, das verschiedene Optiken oder Linsen 27, 28 (usw.) enthält, welche den vom Interferometer 10 einfallenden Strahl an die unterschiedlichen Orte fokussiert. Das Linsenrad 26 wird in Richtung des in Fig. 12 dargestellten Pfeiles durch einen Antrieb 29 in Drehung versetzt, so daß die einzelnen Optiken 27, 28 usw. nacheinander in den Strahlengang gedreht werden. Ein Beispiel für die Linsen, die für die Optiken 27, 28 usw. eingesetzt werden können, sind keilförmige Linsen mit unterschiedlichen Radien, die nicht nur eine axiale Fokusverstellung (Grund unterschiedlicher Brennweite), sondern auch eine laterale Fokusverschiebung bewirken.

Anstatt des Linsenrades kann die Programmoptik 22 natürlich auch deformierbare und/oder verkippbare Spiegel oder Linsen, räumliche Lichtmodulatoren auf Flüssigkristallbasis (sogenannte liquid crystal spacial light modulators, LC-SLM) umfassen, die von einem geeigneten Musterspeicher und/oder -generator angesteuert werden. Die Programmoptik 21 kann schrittweise oder auch kontinuierlich verstellt werden. Im letzteren Fall lassen sich Übergangsphasen von einem Programmschritt zum nächsten, z. B. mit einer momentanen Optikdezentrierung, auch zum Herausmitteln von Speckeln aus überwiegend volumenstreuen Strukturen nutzen, wie es beispielsweise das Volumen der Hornhaut 2 oder der Linse 4 sind.

Die Zusammenfassung der A-Scan-Einzelsignale kann, wie bereits erwähnt, auf verschiedene Art und Weise erfolgen. Auch kann die Zusammenfassung tiefenabhängig gestaltet sein. So kann eine Maximalwertauswahl im Bereich der Linse 4 und eine Mittelung im Bereich der Netzhaut 25 vorgesehen werden. Fig. 12 zeigt weiter noch die Einkopptung des Fixierbildes 12 über den Strahlteiler 11. Falls der dadurch bewirkte Fixationsstimulus durch die Programmoptik 21 gestört wird, kann die Fixation auch nach der Programmoptik eingekoppelt werden oder aber mittels geeigneter Filter vor der Programmoptik geblockt werden. Aufgrund der vergleichsweise schnellen Abfolge der Programmschritte wird für einzelne Programmschritte das Fixationsbild abgeschaltet, was für einen Patienten jedoch nicht wahrnehmbar ist.

Wie bereits erwähnt, erfolgt die Fokuslageveränderung bzw. die Polarisationsänderung nicht synchronisiert zur Aufnahme der A-Scan-Einzelsignale, jedoch vorzugsweise hinreichend langsam gegenüber der Aufnahme eine A-Scan-Einzelsignals, so daß z. B. mehr als die Hälfte der aufgenommenen A-Einzelsignale bei im wesentlichen unveränderter Fokuslage/ Polarisationszustand erfaßt werden.

Die Steuereinrichtung, welche aus den A-Scan-Meßsignalen die gewünschten Teilstrecken im Auge mißt, verwertet also den aktuellen Zustand der Polarisations- oder Fokuslageverstellung nicht. Dennoch ist es, wie bereits angesprochen, möglich, zusätzliche Informationen über die Geometrie der Augenlinse 4 (auch als Kristallinse bezeichnet) aus einer Auswertung der Signale zu gewinnen. Dies ist in Fig. 13 dargestellt. Bei den ermittelbaren Parametern handelt es sich z.B. um die Verkippung der Linse 4, die Dicke der Linse 4 sowie die Radien der posterioren und anterioren Fläche der Linse 4. Diese Informationen können durch eine Auswertung der A-Scan-Einzelsignale gewonnen werden. Jedes A-Scan-Einzelsignal liefert eine Lage Lv der anterioren Linsenfläche sowie eine Lage Lr der posterioren Linsenfläche. Jede solche Messung wird nun in ein Diagramm eingetragen, wie es Fig. 13 zeigt. In Fig. 13 symbolisiert jeder Stern 30 ein Paar aus ermittelten Lagen der anterioren Linsenfläche und der posterioren Linsenfläche, das aus einem A-Scan-Einzelsignal stammt.

Auf der vertikalen Achse ist die Lage Lr der posterioren Linsenfläche aufgetragen, auf der horizontalen Achse die Lage Lv der anterioren Linsenfläche 23

Die aufgetragenen Paare 30 ergeben eine symmetrische Kurve, die symmetrisch zu einer Symmetrieachse 32 liegt.

Das Paar 31, für das sich die maximale Differenz zwischen Lv und Lr ergibt, gibt automatisch die Dicke der Linse an, da sich aus der am meisten anterior liegenden Lage Lv der Linsenvorderfläche 23 und der meisten posterior liegenden Lage Lr der anterioren Linsenfläche 24 automatisch die Linsendicke ergibt.

Die Öffnung der Kurve, d. h. der maximale Abstand 33 zweier Punkte bezogen auf die Symmetrieachse, ist ein Maß für die Verkippung der Linse. Es wird deshalb bzw. aus der Öffnung der Kurve gegenüber der Symmetrieachse 32 die Verkippung der Linse ermittelt. Die Verkippung der Linse ist gleich dem Winkelabstand zwischen optischer Achse und Sehachse des Auges bzw. der Winkel der Linsenäquatorebene zur Sehachse.

Der Abstand zwischen dem Scheitel der sich ergebenden Kurve und einem Schnittpunkt 36, der sich senkrecht zur Symmetrieachse 32 für die gegenüber der Symmetrieachse 32 am weitesten beabstandeten Punkte ergibt, ist in Fig. 12 mit 34 eingetragen und ist proportional zum inversen Radius der anterioren Linsenfläche 23 sowie dem maximalen lateralen Abstand der Fokuspunkte ab.

Analoges gilt für den mit 35 bezeichneten Abstand zwischen dem Scheitel 31 und dem Schnittpunkt 36, der proportional zum inversen Radius der posterioren Linsenfläche 24 und dem maximalen lateralen Abstand ist. Unter Kenntnis des maximalen lateralen Abstandes kann somit aus den Größen 35 und 34 der Radius der anterioren Linsenfläche und der posterioren Linsenfläche ermittelt werden. Hierzu kann z. B. auf ein geeignetes Kennfeld zugegriffen werden, das zuvor aus experimentellen Daten erzeugt wurde. Es sei noch einmal betont, daß es für diese Analyse nicht erforderlich ist, zu wissen, wann der Fokus an welcher Stelle war. Für die beschriebene Analyse ist es lediglich erforderlich zu wissen, welche Paare 30 von Lv und Lr in den A-Scan-Einzelsignale auftraten, aber nicht wann bzw. in welchem A-Scan-Einzelsignal.

Der Ansatz ist auch auf die postoperative Vermessung von Linsenimplantaten (IOL) zur Prüfung von Linsenform und -lage übertragbar und auch auf die Vermessung der Homhautradien, beispielsweise zur Bestimmung von Gullstrand-Verhältnissen. Die Hornhaut entspricht letztendlich auch einer Linse mit gekrümmten Flächen, wobei allerdings das Vorzeichen des posterioren Krümmungsradius gegenüber dem bei der Augenlinse geändert ist. Dadurch erscheint dann die in Figur 13 dargestellte Kurve im Fall einer Hornhautvermessung vertikal gespiegelt, ist aber analog auswertbar.

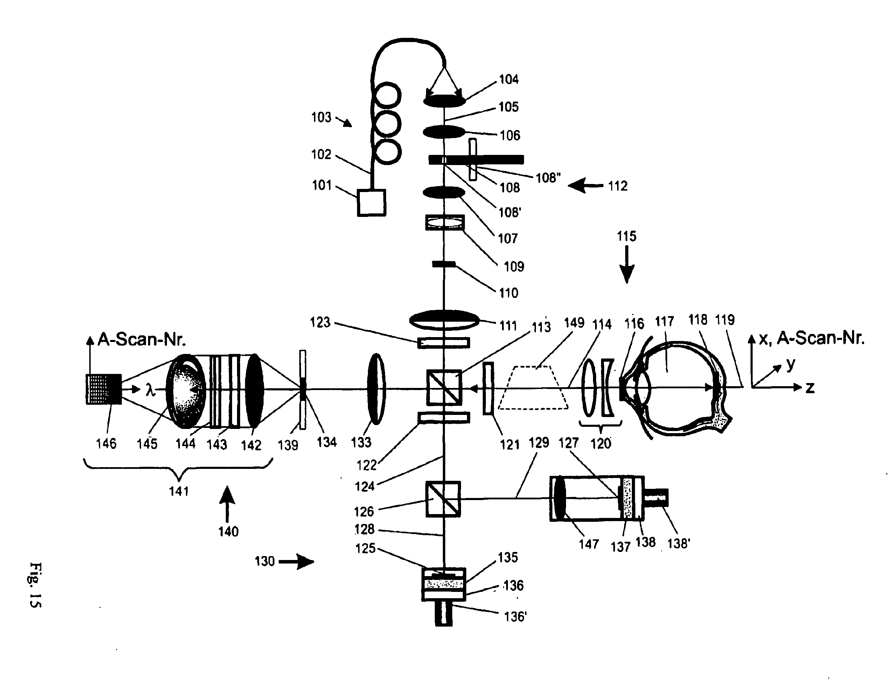

Die Figuren 15 bis 18 präsentieren alternative erfindungsgemäße Anordnungen polarisationsoptischer (Verwendung einer polarisationsoptischen doppelbrechenden Optik zur Objektbeleuchtung) oder diffraktionsoptischer (Verwendung einer diffraktionsoptischen doppelbrechenden Optik zur Objektbeleuchtung) Parallel-FD-KKI.

In Figur 15 ist ein erfindungsgemässer Strahlengang dargestellt, der zur gleichzeitigen Erfassung zweier Messfelder eine polarisationsoptische doppelbrechende Optik (111) benutzt. 101 ist eine Lichtquelle mit kurzer Kohärenzlänge jedoch voller Raumkohärenz, beispielsweise eine Superlumineszenzdiode oder ein breitbandig im transversalen Monomodenbetrieb arbeitender Laser. Die Lichtleitfaser 102 leitet das von 101 emittierte Licht durch den Polarisations-Controller 103 zu der Kollimationsoptik 104. Das aus dieser austretende Lichtbündel 105 beleuchtet das Michelson-Interferometer. Die Optik 106 bildet mit der Optik 107 ein telezentrisches Optikpaar, in dessen gemeinsamer Brennebene sich die Öffnung 108' eines um seine Achse 108" rotierenden Choppers 108 befindet. Der Chopper 108 definiert die Belichtungszeiten für das CCD-Array 146. Die Optik 107 kollimiert das hinter dem Chopper divergierende Lichtbündel wieder zu einem Parallelbündel 105. Dieses trifft auf die Zylinderoptik 109 mit horizontaler Zylinderachse und wird von dieser in einen Linienfokus 110 fokussiert. Der Linienfokus 110 wird von der doppelbrechenden Optik 111 in zwei unterschiedliche Bildweisen abgebildet. Die doppelbrechende Optik 111 kann entweder mittels Polarisationsoptik oder mittels diffraktiver Optik realisiert werden.

Der auf Polarisationsoptik beruhende Strahlengang der Figur 15 benutzt polarisationsoptische doppelbrechende Linsen. Solche Linsen können aus Kristallen zweier verschiedener Doppelbrechungstypen (positiv und negativ) realisiert werden, wobei deren Kristallachse in der Linsenebene liegt. Im Beispiel von negativ doppelbrechendem Kalkspat (Brechungsindex des ordentlichen Strahis $n_o$ > Brechungsindex des außerordentlichen Strahls $n_{ao}$) als Werkstoff für eine doppelbrechende Linse, wird in der Ebene zur Kristallachse schwingendes Licht stärker gebrochen als Licht, das normal zur Kristallachse schwingt. Bei positiv doppelbrechenden Kristallen, wie Quark, wird das in der Ebene der Kristallachse schwingende Licht schwächer gebrochen als Licht, das normal zur Kristallachse schwingt. Eine doppelbrechende Linse hat daher für zwei normal zueinander schwingende Lichtwellen zwei unterschiedliche Brennweiten. Im Folgenden wird die Kristallachse von 111 normal zur Zeichenebene orientiert angenommen und Kalkspat als Linsenmaterial. Eine Abbildung des Linienfokus 110 in zwei unterschiedliche Bildweiten wird hier mit Hilfe des Polarisations-Controllers 103 gesteuert. Mit dessen Hilfe kann man die Schwingungsebene des Lichts im Beleuchtungsstrahl 105 so einstellen, beispielsweise unter 45° zur Zeichenebene, dass dieses sowohl eine Komponente parallel als auch normal zur Kristallachse der doppelbrechenden Optik 111 besitzt. Diese beiden normal zueinander schwingenden Komponenten werden - entsprechend den zugehörigen Brennweiten der doppelbrechenden Optik 111 - den Linienfokus 110 in unterschiedliche Bildweiten abbilden.

Der Messstrahl 114 wird von den am Strahlteiler 113 reflektierten Abbildungsstrahlen gebildet. Der Messstrahl 114 erzeugt im Messarm 115 je ein Bild 116 des Linienfokus 110 in der Eintrittspupille des Auges und ein Bild 118 im Fundus des Auges 117. Hierzu wird die doppelbrechende Optik 111 beispielsweise bei Verwendung von negativ doppelbrechendem Kalkspat so ausgelegt, dass der ordentliche Teil des Strahls 105 das Bild 116 an einer Stelle der optischen Achse 119 erzeugt, wo das Auge mit seiner Eintrittspupille positioniert werden soll. Ferner kann man die doppelbrechende Linse 110 beispielsweise so aufstellen und dimensionieren, dass der ausserordentliche Teil des Strahls 105 den Linienfokus 110 zunächst nach Unendlich abbildet, so dass er von einem emmetropen Auge im entspannten Zustand auf der Netzhaut zur Abbildung kommt. Die Abbildung des zunächst nach Unendlich abgebildeten Linienfokus auf die Netzhaut kann bei ametropen Augen unter Zuhilfenahme einer die Fehlsichtigkeit kompensierenden Zoom-Optik 120 erreicht werden.

Eine Optimierung der Anpassung der Messstrahl-Intensitäten in den zwei die Abbildungen des Linienfokus realisierenden Lichtbündeln an die unterschiedlich starken Reflektivitäten der betreffenden Augenstrukturen ist vorteilhaft. Durch Einstellung der Schwingungsebene im Strahl 105 mit Hilfe des faseroptischen Polarisations-Controllers 103 relativ zur optischen Kristallachse der Optik 111 kann die Aufteilung der Intensitäten auf die Komponenten parallel als auch normal zur Kristallachse eingestellt werden. Alternativ kann dies auch durch Drehen der Polarisationebene mittels drehbaren Polarisators oder einer λ/2-Platte, beispielsweise zwischen den Optiken 104 und 106 angeordnet, erfolgen.

Der Referenzarm 130 mit dem Referenzstrahl 124 wird durch die vom Strahlteiler 113 transmittierten Abbildungsstrahlen aus dem Beleuchtungsarm 112 gebildet. Die doppelbrechende Optik 111 erzeugt auch im Referenzarm je ein Bild des Linienfokus 110 in unterschiedlichen Bildweiten. Ein Strahlteiler 126 teilt ferner den Referenzstrahl 124 in einen Teil-Referenzstrahl 128, angepasst an die Messarmlänge zur Eintrittspupille des Auges, und einen Teil-Referenzstrahl 129, mit der im Abstand ihrer Brennweite vor dem Reflektor 137 angeordneten Optik 147. Der Teil-Referenzstrahl 129 ist an die Messarmlänge zum Fundus des Auges angepasst. Die Referenzspiegel 135 und 137 (mit Optik 147) sind auf elektrisch steuerbaren Stelltischen 136 und 138 mit Stellmotoren 136' und 138' montiert. (In jedem der 2 Teil-Referenzarme entsteht noch ein zweites Linienfokus-Bild, das jedoch letztlich defokussiert auf den Spalt 139 abgebildet und so unterdrückt wird.)

Eine weitere Optimierung erreicht man durch eine flexible Verteilung der Strahlintensitäten auf Interferometer-Messarm 115 und Interferometer-Referenzarm 130, indem man 113 als Polarisations-Strahlteiler ausbildet und davor eine drehbare λ/2-Platte 123 setzt. Drehen der λ/2-Platte erlaubt eine kontinuierliche Veränderung der Balance der Intensitäten in Messarm 15 und Referenzarm 130.

Eine Optimierung durch Reduzierung der Licht-Verluste von Messarm und Referenzarm kann am Polarisations-Strahlteiler 113 durch λ/4-Platten 121 und 122 (unter 45° zur Zeichenebene) erreicht werden. Werden diese λ/4-Platten so orientiert, dass sie zirkular polarisiertes Licht erzeugen, bewirken sie für hin- und zurück-laufendes Licht aus den jeweiligen Interferometerarmen (115 und 130) eine Drehung der Schwingungsebene um 90°: Das vom Messarm 115 zurück kommende Licht wird ohne Reflexions-Verluste durch den Polarisationsstrahlteiler 113 in den Spektrometerarm 141 transmittiert, das vom Referenzarm 130 zurück kommende Licht wird ohne Transmissions-Verluste durch den Polarisationsstrahlteiler 113 in den Spektrometerarm 141 reflektiert.

Im Spektrometerarm 140 erzeugt die doppelbrechende Optik 133 sekundäre Abbildungen 134 der Bilder 116, 118, 125 und 127 des Linienfokus 110. Wenn die Optiken 111 und 133 gleiche Brennweiten haben und symmetrisch um die Teilerebene des Strahlteilers 113 angeordnet werden, liegt ein besonders überschaubarer Fall vor: Die Sekundärbilder der Bilder 116, 118, 125 und 127 entstehen in derselben Größe an derselben Stelle 134. An der Stelle des sekundären Linienfokusbilds 134 befindet sich der Eintrittspalt 139 (rechteckige Öffnung, in die Zeichenebene geklappt gezeichnet) des Spektrometers 141. Dieser eliminiert parasitäre aus dem Interferometer reflektierte Lichtanteile.

Die Kollektoroptik 142 des Spektrometers bildet das sekundäre Linienfokusbild über das Beugungsgitter 144 mit Hilfe der Spektrometeroptik 145 auf das zweidimensionale Detektor-Array 146 ab. 143 ist ein Analysator, der die Interferenzfähigkeit der zusammengehörigen Mess- und Referenzlichtbündel sicherstellt. Man kann diesen auch drehbar ausbilden, was eine weitere Anpassung der Intensitäten der Interferometer-Strahlen ermöglicht. Das Beugungsgitter dispergiert das sekundäre Linienfokusbild wellenlängenabhängig entlang der λ-Koordinate des zweidimensionalen Detektor-Arrays 46.

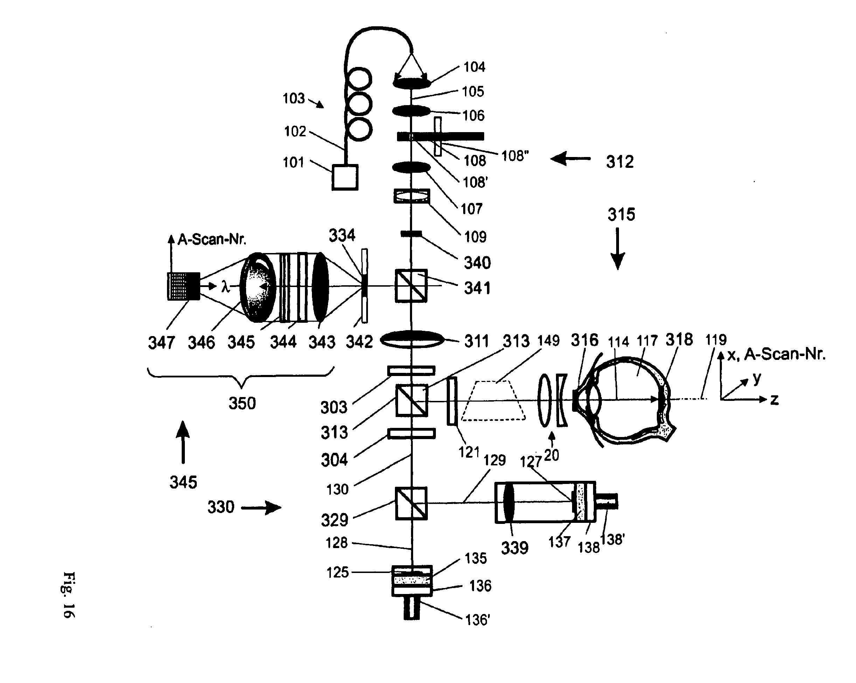

Die Beugungsrichtung zeigt aus der Zeichenebene heraus; Spektrometeroptik 145 und Detektor-Array 146 sind daher - auch in den Figuren 16, 17 und 18 - um eine horizontale Achse aus der Zeichenebene heraus nach unten gekippt angedeutet. Die Scan-Nr. der Figur 14 entspricht hier der x-Koordinate im Auge. Man erhält sogleichzeitig - für in transversaler Richtung benachbarte A-Scans aus dem Spektrum des zugehörigen Interferometer-Signals als Eingangsdatensatz mittels FT die Tiefenstruktur entlang der z-Koordinate. Die in Figur 15 skizzierte Anordnung hat den Vorteil, dass sich zwischen dem Auge und dem Detektor nur 1 Strahlteiler (113) befindet. Das macht es leicht, dessen sensitvitätsbegrenzenden Einfluss zu beheben. Nachteilig ist hingegen das Erfordernis der polarisationsoptischen doppelbrechenden refraktiven Optiken (111 und 133), weil diese auf der Verwendung ungewöhnlicher Werkstoffe (beispielsweise Kalkspat) basieren, die schwer mit hoher Präzision bearbeitbar oder käuflich sind.

Im Übrigen kann man im Strahlengang nach Figur 15 anstelle der polarisationsoptischen doppelbrechenden Optiken 111 und 133 auch diffraktive doppelbrechende Optiken mit den entsprechenden Brennweiten benutzen. Wegen der geringen optischen Qualität doppelbrechender Optiken ist es jedoch ganz allgemein sinnvoll, diese nicht häufiger als unbedingt erforderlich einzusetzen.

Figur 16 präsentiert eine erfindungsgemäße Alternative, die zur gleichzeitigen Erfassung zweier Messfelder auf der Verwendung eines einzigen doppelbrechende Elements, hier einer diffraktiven doppelbrechenden Optik, beruht.

Wie in der Figur 15 ist auch im Strahlengang der Figur 16 die Position 101 eine Lichtquelle mit kurzer Kohärenzlänge jedoch voller Raumkohärenz, beispielsweise eine Superlumineszenzdiode oder ein breitbandig im transversalen Monomodenbetrieb arbeitender Laser. Die Lichtleitfaser 102 leitet das von 101 emittierte Licht durch den Polarisations-Controller 103 zu der Kollimationsoptik 104. Der faseroptische Polarisations-Controller 103 erlaubt ein Drehung der Polarisationsebene des Lichtbündels im Interferometer und damit bei Verwendung eines Polarisationsstrahlteilers 313 eine Optimierung der Lichtverteilung zwischen Messstrahl 114 und Referenzstrahl 130. Das aus der Kollimationsoptik 104 austretende Lichtbündel 105 beleuchtet das Interferometer. Die Optik 106 bildet mit der Optik 107 ein telezentrisches Optikpaar in dessen gemeinsamer Brennebene sich die Öffnung 108' eines Choppers 108 mit der Drehachse 108" befindet. Der Chopper 108 definiert die Belichtungszeiten für das CCD-Array 347. Die Optik 107 kollimiert das hinter dem Chopper 108 divergierende Lichtbündel wieder zu einem Parallelbündel. Dieses trifft auf die Zylinderoptik 109 und wird von dieser in einen Linienfokus 340 fokussiert. Nach dem Linienfokus 340 durchsetzt das Lichtbündel 105 den Strahlteiler 341. Der Linienfokus 340 wird von der doppelbrechenden Optik 311 in zwei unterschiedliche Bildweiten abgebildet. Die doppelbrechende Optik 311 ist hier eine diffraktive Optik (kann jedoch auch eine polarisationsoptische sein).

Diffraktive Optiken können mehrere Brennweiten haben. (Deren Werte hängen von der radialen Raumfrequenz der in diesen Optiken benutzten Fresnel-Linsen und FresnelPhasenplatten ab.) Hier wird vorzugsweise eine diffraktive Optik (311) mit nur 2 Brennweiten benutzt. Weitere Brennweiten würden nur Licht aus dem Strahlengang ungenutzt abzweigen. Entsprechend wird der Linienfokus 340.in 2 unterschiedliche Bildweiten abgebildet.

Den Messstrahl bilden die vom Strahlteiler 313 reflektierten Abbildungsstrahlen. Diese erzeugen im Messarm 315 je ein Bild 316 des Linienfokus 340 in der Eintrittspupille des Auges 117 und ein Bild 318 im Fundus des Auges. Hierzu wird die doppelbrechende Optik 311 so ausgelegt, ein erster gebeugter Teil des Strahis 105 das Bild 316 an einer Stelle der optische Achse 119 erzeugt, wo das Auge mit seiner Eintrittspupille positioniert werden soll. Die doppelbrechende Optik 311 wird außerdem so aufgestellt und dimensioniert, dass der zweite gebeugte Teil des Strahis 105 den Linienfokus 340 zunächst nach Unendlich abbildet, so dass 340 in einem emmetropen Auge im entspannten Zustand auf der Netzhaut zur Abbildung kommt (318). Die Abbildung des zunächst nach Unendlich abgebildeten Linienfokus kann bei ametropen Augen unter Zuhilfenahme einer die Fehlsichtigkeit kompensierenden Zoom-Optik 120 erreicht werden.

Der Referenzarm 330 wird durch die vom Strahlteiler 313 transmittierten Abbildungsstrahlen aus dem Beleuchtungsarm 312 gebildet. Die doppelbrechende Optik 311 erzeugt auch im Referenzarm je ein Bild des Linienfokus 340 in unterschiedlichen Bildweiten. Ein Strahlteiler 329 teilt ferner den Referenzstrahl 130 in einen Teil-Referenzstrahl 128, angepasst an die Messannlänge zur Eintrittspupille des Auges, und einen Teil-Referenzstrahl 129, angepasst an die Messannlänge zum Fundus des Auges. Die Referenzspiegel 135 und 137, ebenso die Optik 339, deren Brennweite ihrem Abstand von dem Referenzspiegel37 entspricht, sind auf elektrisch steuerbaren Stelltischen 136 und 138 mit Stellmotoren 136' und 138' montiert. (Auch hier entsteht in jedem der 2 Teil-Referenzarme noch ein zweites Linienfokus-Bild, das jedoch letztlich defokussiert auf den Spalt 139 abgebildet und so unterdrückt wird.)

Im Spektrometerarm 340 erzeugt die doppelbrechende Optik 311 sekundäre Abbildungen 334 der Bilder 316, 318, 125 und 127 des Linienfokus 340. Diese Sekundärbilder der Bilder 316, 318, 125 und 127 entstehen in derselben Größe an derselben Stelle 334. An der Stelle des sekundären Linienfokusbilds 334 befindet sich der Eintrittspalt 342 (rechteckige Öffnung in die Zeichenebene geklappt gezeichnet) des Spektrometers 350. Dieser eliminiert parasitäre, aus dem Interferometer reflektierte, Lichtanteile. Die Kollektoroptik 343 des Spektrometers 350 bildet das sekundäre Linienfokusbild weiter über das Beugungsgitter 345 mit Hilfe der Spektrometeroptik 346 auf das zweidimensionale Detektor-Array 347 ab. 344 ist ein Analysator, der optimale Interferenzfahigkeit der zusammengehörigen Mess- und Referenzlichtbündel sicherstellt. Das Beugungsgitter 345 dispergiert das Linienfokusbild auf dem Detektor-Array 347 wellenlängenabhängig entlang der A-Koordinate. Die Scan-Nr. der Figur 14 entspricht auch hier der x-Koordinate im Auge. Man erhält hier gleichzeitig, ebenso wie nach der Anordnung der Figur 15, alle Spektren für die in x-Richtung benachbarten AScan Interferometer-Signale. Diese bilden den Eingangsdatensatz für die PT zur Berechnung der Tiefenstruktur entlang der z-Koordinate.

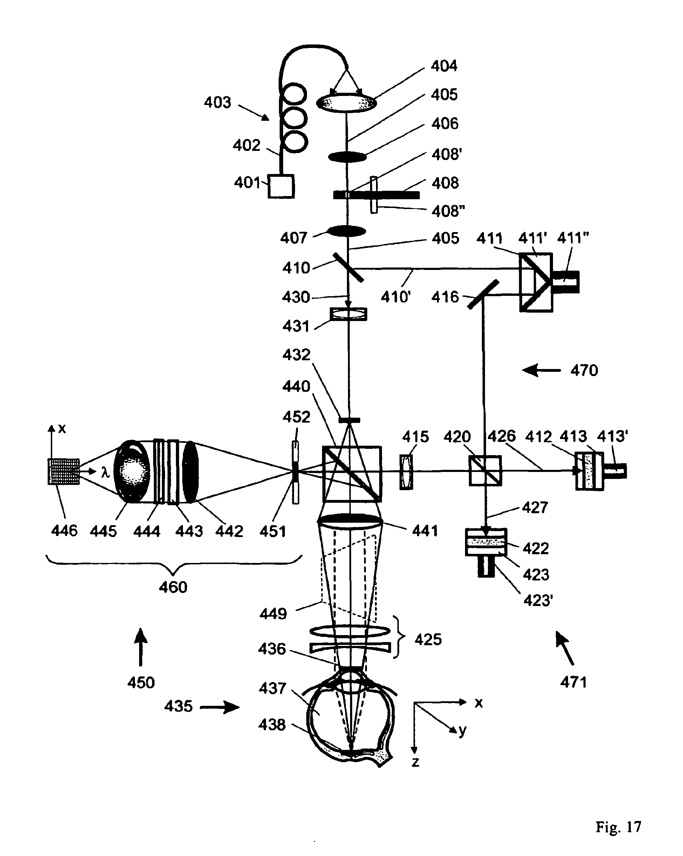

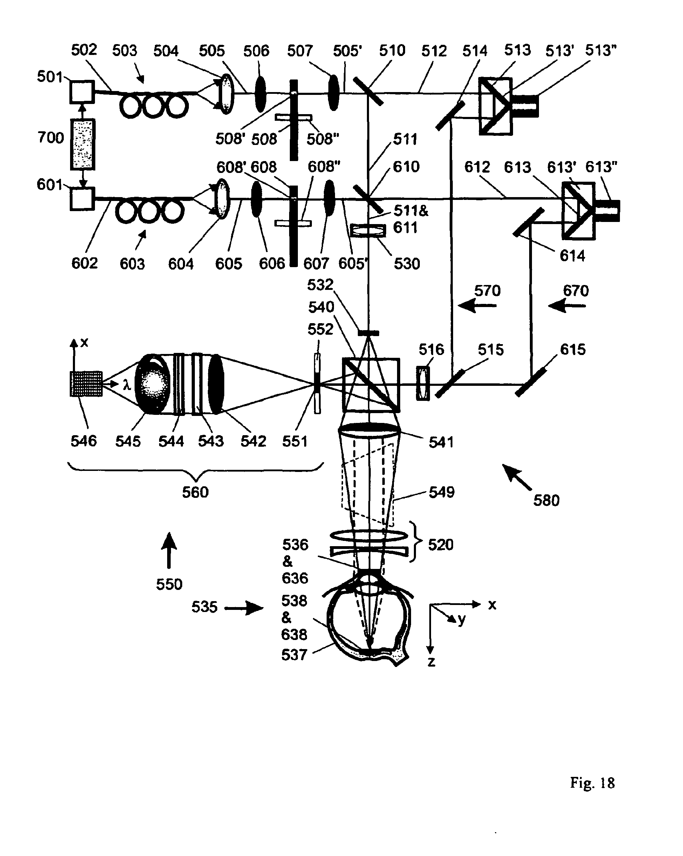

Die eingangs aufgelisteten Erfordernisse zur Gewinnung optimaler Signale werden von den bisher beschriebenen Anordnungen unterschiedlich angenähert: während die Anordnung nach Figur 15 zwei doppelbrechende Optiken und einen Strahlteiler benutzt, kommt die Anordnung nach Figur 16 mit einer doppelbrechenden Optik aus, benötigt jedoch 2 Strahlteiler zwischen dem Auge und dem Spektrometer. Während Strahlteiler immer zu Lichtverlusten und/oder zusätzlichem Aufwand zur Vermeidung derselben führen, sind doppelbrechende Optiken hoher Qualität schwierig herzustellen und teuer. Günstigere Interferometer ergeben unter diesem Aspekt Strahlengänge aus Kombinationen von Mach-Zehnder und Michelson-Interferometer-Anordnungen, wie in den Figuren 17 und 18 dargestellt, wobei dort der Mach-Zehnder Teil den Beleuchtungs- und Referenzarm des Michelson-Interferometers bildet.

Figur 17 zeigt eine KKI-Anordnung mit nur einer einzigen doppelbrechenden Optik und nur einem einzigen Strahlteiler zwischen Auge und Spektrometer. Der Strahlengang ist ein KKInterferometer mit einem Mach-Zehnder ähnlichen Referenzstrahlengang mit optischem Auszug zur Weglängenanpassung. 401 ist eine Lichtquelle mit kurzer Kohärenzlänge jedoch voller Raumkohärenz, beispielsweise eine Superlumineszenzdiode oder ein breitbandig im transversalen Monomodenbetrieb arbeitender Laser. Die Lichtleitfaser 402 leitet das von 401 emittierte Licht durch den Polarisations-Controller 403 zu der Kollimationsoptik 404. Das aus dieser austretende Lichtbündel 405 beleuchtet das Interferometer. Die Optik 406 bildet mit der Optik 407 ein telezentrisches Optikpaar, in dessen gemeinsamer Brennebene sich die Öffnung 408' eines um seine Achse 408" rotierenden Choppers 408 befindet. Der Chopper 408 definiert die Belichtungszeiten für das CCD-Array 446. Die Optik 407 kollimiert das hinter dem Chopper divergierende Lichtbündel wieder zu einem Parallelbündel 405. Dieses trifft auf den Strahlteiler 410, der daraus ein erstes Referenzbündel 410' reflektiert. Der den Strahlteiler 420 transmittierende Teil des Strahls 405 bildet den Messstrahl 430 und trifft auf die Zylinderlinse 431 mit hier horizontal angenommener Zylinderachse, die den Messstrahl in einen horizontalen Linienfokus 432 fokussiert.

Das vom Linienfokus 432 in der Vertikalen divergierende Lichtbündel durchsetzt den Strahlteiler 440 und wird von der doppelbrechenden Optik 441 in zwei unterschiedliche Bildweiten abgebildet. Diese doppelbrechende Optik kann, wie bereits an den Beispielen der Figuren 15 und 16 beschrieben, entweder mittels polarisationsoptischer doppelbrechender Linsen oder mittels diffraktiver doppelbrechender Optiken so realisiert werden, dass der Messstrahl 430 im Messarm 435 je ein Bild 436 des Linienfokus 432 in der Eintrittspupille des Auges und ein Bild 438 im Fundus des Auges 437 erzeugt. Die Abbildung des zunächst nach Unendlich abgebildeten Linienfokus auf den Fundus kann bei ametropen Augen unter Zuhilfenahme einer die Fehlsichtigkeit kompensierenden Zoom-Optik 425 erreicht werden.

Das von den Linienfokus-Bildern 436 und 438 remittierte Licht wird von der doppelbrechenden Optik 441 über den Strahlteiler 440 in den Spektrometerarm 450 gespiegelt und erzeugt im Falle einer polarisationsoptischen doppelbrechenden Optik zwei sekundäre Bilder an der Stelle 451. Im Falle einer diffraktiven doppelbrechenden Optik 441 erzeugen beide primären Bilder 436 und 438 je zwei sekundäre Bilder, also entstehen insgesamt vier Bilder; von diesen 4 Bildern werden jedoch nur zwei an der Stelle 451 fokussiert, während die anderen beiden dort defokussiert abgebildet werden, also mittels der dort aufgestellten Spaltblende 452 des Spektrometers (deren rechteckige Öffnung in die Zeichenebene geklappt angedeutet ist) weitgehend unterdrückt werden können.

Referenzstrahlen und Messstrahlen bilden im folgenden ein Mach-Zehnder ähnliches Interferometer: Der Referenzstrahl 410' wird vom Strahlteiler 410 aus dem Strahl 405 gespiegelt und trifft auf einen aus 410, 411 und 416 gebildeten optische Auszug: Der Retroreflektor 411 ist auf einem Verschiebetisch 411' montierten und von einem Motor 411", angetrieben. Der 90°-Spiegel 416 spiegelt den Referenzstrahl auf das in einer Ecke des Mach-Zehnder Interferometers 470 positionierte Michelson-Interferometer 471. Der Strahlteiler 420 trennt die beiden Referenzstrahlen 426 und 427. Die Referenzspiegel 412 und 422 sind auf elektrisch steuerbaren Stelltischen 413 und 423 mit Stellmotor 413' und mit Stellmotor 423' montiert, die eine stufenlose Veränderung der optischen Weglänge dieser Referenzbündel relativ zueinander erlauben. Die von den Referenzspiegeln reflektierten Referenzstrahlen 426 und 427 treffen auf die Zylinderlinse 415, die sie durch den Strahlteiler 440 hindurch in den Linienfokus 451 im Spektrometerarm 450 fokussiert.

Der (nun von insgesamt 4 Strahlen gebildete) Linienfokus 451 befindet sich in der Eintrittspupille des Spektrometers 460 mit dem Eintrittspalt 452. Die Kollektoroptik 442 des Spektrometers bildet das sekundäre Linienfokusbild über das Beugungsgitter 444 mit Hilfe der Spektrometeroptik 445 auf das zweidimensionale Detektor-Array 446 ab. 443 ist ein Analysator, der die Interferenzfähigkeit der verschiedenen Mess- und Referenzbündel gewährleistet. Das Beugungsgitter dispergiert dieses Linienfokusbild wellenlängenabhängig entlang der $\lambda$-Koordinate des Detektor-Arrays. Die x-Koordinate des Detektor-Array 446 entspricht der x-Koordinate im Auge und der Scan-Nr. der Figur 14. Man erhält somit gleichzeitig - für benachbarte A-Scans aus dem Spektrum des zugehörigen Interferometer-Signals als Eingangsdatensatz mittels FT die Tiefenstruktur entlang der z-Koordinate.

Man kann den Strahlteiler 410 auch weglassen und die Referenzstrahlen mit Hilfe des am Strahlteiler 440 reflektierten Teils des Messstrahts gewinnen. Die Zylinderlinse 415 muss virtuell um die Spiegelfläche des Strahlteilers 440 im Abstand ihrer Brennweite vom Linienfokus 432 aufgestellt werden. Dann entfallen auch die Reflektoren 411 und 416. Allerdings sind dann die Intensitäten der Referenzstrahlen nicht mehr ganz unabhängig von jenen der Messstrahlen.

Figur 18 präsentiert einen KKI-Strahlengang von gleichzeitig zwei KK-FD Interferometern in einem: Zwei separate Lichtquellen, 501 und 601, mit kurzer Kohärenzlänge jedoch voller Raumkohärenz, wie Superlumineszenzdioden oder breitbandig im transversalen Monomodenbetrieb arbeitende Laser, beleuchten zwei separate Mach-Zehnder Strahlengänge 570 und 670 mit einem teilweise gemeinsamen Arm. Beide Strahlengänge münden in einem Michelson Interferometer 580 und beleuchten dort letztlich dasselbe Messobjekt und einen gemeinsamen Spektrometerarm 550.

Die Lichtleitfaser 502 (602) leitet das von den KK-Lichtquellen 501 (601) emittierte Licht durch den Polarisations-Controller 503 (603) zu der Kollimationsoptik 504 (604). 700 ist eine von aussen, beispielsweise per PC steuerbare, Stromversorgung der Lichtquellen 501 und 601. Das Lichtbündel 505 (605) beleuchtet das Interferometer. Die Optiken 506 und 606 bilden mit den Optiken 507 und 607 telezentrische Optikpaare, in deren gemeinsamer Brennebene sich die Öffnungen 508' und 608' um ihre Achsen 508" und 608" rotierende Chopper 508 und 608 befinden. Diese Chopper definieren die Belichtungszeiten für das CCD-Array 546. Die Optiken 507 und 607 kollimieren die hinter diesen Choppem divergierenden Lichtbündel wieder zu Parallelbündein (505' und 605'). Diese treffen auf die Strahlteiler 510 und 610, die diese Strahlen in Messstrahlen 511 und 611 sowie Referenzstrahlen 512 und 612 aufspalten.

Die zwei Messstrahlen 511 und 611 werden von der doppelbrechenden Optik 530 in den Linienfokus 532 fokussiert. Die zwei nun vom Linienfokus 532 (vertikal) divergierenden Lichtbündel durchsetzen den Strahlteiler 540 und werden von der doppelbrechenden Optik 541 in zwei unterschiedliche Bildweiten abgebildet. Diese doppelbrechende Optik kann, wie bereits an den obigen Beispielen beschrieben, entweder mittels polarisationsoptischer doppelbrechender Linsen oder mittels diffraktiver doppelbrechender Optiken so realisiert werden, dass die Messstrahlen 511 und 611 je ein Bild 536 und 636 des Linienfokus 532 in der Eintrittspupille des Auges und je ein Bild 538 uns 638 im Fundus des Auges 537 erzeugen. Der zunächst nach Unendlich abgebildete Linienfokus kann bei ametropen Augen unter Zuhilfenahme einer die Fehlsichtigkeit kompensierenden Zoom-Optik 520 auf den Fundus abgebildet werden.

Das von den Linienfokus-Bildern 536, 636 sowie 538 und 638 remittierte Licht wird von der doppelbrechenden Optik 541 über den Strahlteiler 540 in den Spektrometerarm 550 gespiegelt und erzeugt im Falle einer polarisationsoptischen doppelbrechenden Optik 541 zwei sekundäre Bilder an der Stelle 551. Im Falle einer diffraktiven doppelbrechenden Optik 541 erzeugen beide primären Bilder 536 und 538 je zwei sekundäre Bilder, also entstehen insgesamt vier Bilder, von diesen 4 Bildern werden jedoch nur zwei an der Stelle 551 fokussiert, während die anderen beiden dort defokussiert abgebildet werden, also mittels der dort aufgestellten Spaltblende 552 des Spektrometers (deren rechteckige Öffnung in die Zeichenebene geklappt angedeutet ist) weitgehend unterdrückt werden können.

Die die Strahlteiler 510 und 610 transmittierenden Teile der Strahlen 505 und 605 bilden die Referenzstrahlen.

Das am Strahlteiler 510 transmittierte Referenzbündel 512 trifft auf einen aus Dachkantspiegel 513 und 45°-Spiegel 514 bestehenden optischen Auszug. Der Dachkantspiegel 513 ist auf einem elektrisch steuerbaren Stelltisch 513' mit Stellmotor 513" montiert, der eine stufenlose Veränderung der optischen Weglänge des Referenzbündels 512 erlaubt. Dieses Referenzbündel 512 wird über den 45°-Strahlteiler 515 zur Zylinderlinse 516 reflektiert, die es durch den Strahlteiler 540 hindurch in den Linienfokus 551 fokussiert.

Das am Strahlteiler 610 transmittierte Referenzbündel 612 trifft ebenfalls auf einen optischen Auszug, bestehend aus Strahlteiler 610, Dachkantspiegel 613 und 45°-Spiegel 614. Der Dachkantspiegel 613 ist auf einem elektrisch steuerbaren Stelltisch 613' mit Stellmotor 613" montiert. Dieses Referenzbündel wird über den 45°-Strahlteiler 615 zur Zylinderlinse 516 reflektiert, die es durch den Strahlteiler 540 hindurch ebenfalls in den Linienfokus 551 im Spektrometerarm 550 fokussiert.

Die optischen Wegdifferenzen der Referenzstrahlen zu den Messstrahlen werden beispielsweise bei der Augen-Achslängenmessung so eingestellt, dass je ein separates Messfeld aus dem Corneabereich Eintrittspupille und ein Messfeld aus dem Fundus abgebildet werden.

Der nun von 2 Messstrahlen und 2 Referenzstrahlen gebildete Linienfokus 551 befindet sich in der Eintrittspupille des Spektrometers 560 mit dem Eintrittspalt 552. Die Kollektoroptik 542 des Spektrometers bildet das sekundäre Linienfokusbild über das Beugungsgitter 544 mit Hilfe der Spektrometeroptik 545 auf das zweidimensionale Detektor-Array 546 ab. 543 ist ein Analysator, der die Interferenzfähigkeit dieser Strahlen sicherstellt. Das Beugungsgitter dispergiert dieses Linienfokusbild wellenlängenabhängig entlang der A-Koordinate des Detektor-Arrays. Die x-Koordinate des Detektor-Array 546 entspricht der x-Koordinate im Auge und der Scan-Nr. der Figur 14. Man erhält somit mittels FT - gleichzeitig - für mehrere benachbarte A-Scans aus dem Spektrum des zugehörigen Interferometer-Signals als Eingangsdatensatz die Tiefenstruktur entlang der z-Koordinate.

Der Vorteil dieser allerdings 2 Lichtquellen erfordernden Anordnung liegt darin, beide Messfelder völlig unabhängig voneinander gestalten zu können. Durch entsprechende Einstellung der optischen Längen der Referenzstrahlen mit Hilfe der optischen Auszüge können die beiden Messfelder beliebig positioniert werden, es können auch unterschiedliche Wellenlängen benutzt werden, um die Eindringtiefen in das Auge zu optimieren und die beiden Messfelder können zur Signal-Identifizierung mittels der Stromversorgung 700 elektrisch sehr schnell aus- und eingeschalten oder in ihrer Helligkeit und mit unterschiedlichen Frequenzen und Signalformen moduliert werden. Die bereits im Zusammenhang mit der Figur 15 beschriebenen Optimierungen können auch in den Anordnungen nach den Figur 16, 17 und 18 ausgeführt werden: So können beispielsweise bei auf Polarisationsoptik beruhenden doppelbrechenden Optiken 441 und 541 mit Hilfe der Polarisations-Controller die Intensitäten der zwei Messstrahlen relativ zueinander variiert werden.

Wie eingangs erwähnt, werden mit Hilfe der zwei Referenzstrahlen zwei Messfelder in unterschiedlicher Tiefe realisiert. Diese fallen als Ergebnis der FT des Spektrometers-Signals gleichzeitig an. Damit tritt das Problem auf, die Signale der zwei Messfelder zu erkennen und zu unterscheiden. Hierzu gibt es mehrere Möglichkeiten. Die Standardmethode besteht darin, wie schon erwähnt, unterschiedliche optische Wegdifferenzen zwischen den zusammengehörigen Linienfoki in den Mess- und Referenzstrahlen, also hier beispielsweise wie in der Figur 17 skizziert, zwischen Licht aus 436 und dem Lichtbündel 426 einerseits, sowie zwischen Licht aus 438 und dem Lichtbündel 427 andererseits, einzustellen. Dann werden die zugehörigen Messfelder in unterschiedlichen z-Positionen, wie in der Figur 14 dargestellt, rekonstruiert.

Diese Strategie ist nicht immer erfolgreich; beispielsweise versagt sie häufig in der Anfangsphase einer Messung, wenn durch entsprechende Positionierung des Probanden erste rekonstruierte A-Scan Signale erst einmal gefunden werden müssen. Hierzu ist es vorteilhaft, die einzelnen A-Scan Signale identifizieren zu können. Das ist auf verschiedene Weisen möglich. Beispielsweise kann man durch periodische harmonische Bewegung eines Referenzspiegels (125 oder 127 in den Anordnungen der Figuren 15 und 16) oder der optischen Auszüge in den Anordnungen der Figuren 17 und 18 durch entsprechende Steuerung der zugehörigen Antriebe eine periodische Bewegung der rekonstruierten A-Scan Signale entlang der z-Position (Fig. 14) erreichen. Anstelle einer harmonischen Bewegung können auch anharmonische Bewegungsmuster, beispielsweise sägezahnartige, zur Identfizierung der A-Scan Signale benutzt werden. Neben der angedeuteten einfachen periodischen Bewegung eines Spiegels können beide Referenzspiegel auch gegenläufig bewegt werden oder mit unterschiedlichen Periodendauern. Schließlich kann man durch sehr schnelle Bewegung das eine oder das andere A-Scan Signal auch unsichtbar machen.

Bei Erkrankungen im Fovea-Bereich oder an der Comea-Oberfläche kann es sinnvoll sein, die azimutale Orientierung des Linienfokus im Auge zu verändern. Dies ist durch die Verwendung von Bildrotatoren, wie beispielsweise einem Dove-Prisma (149, 449, 549) oder anderen Reversionsprismen, wie dem Prisma von Abbe/König, dem Prisma von SchmidtlPechan oder dem aus drei Prismen bestehenden Prismenumkehrsystem nach Uppendahl vor dem Auge möglich.

Es sei noch erwähnt, dass anstelle des Polarisations-Controllers in allen Anordnungen im Beleuchtungsarm, beispielsweise nach den Kollimationsoptiken (104, 404, 504, 604) auch rotierende $\lambda/2$-Platten nach einem geeignet orientierten Polarisator und andere zum Stand der Technik gehörende Methoden zum Drehen der Polarisationsebene eines Lichtstrahls benutzt werden können.

Schließlich kann man die Interpretation der den Längenmessungen zugrunde liegenden Augenstrukturen noch dadurch verbessern, dass man die Summen-A-Scan Signale aus zueinander parallel versetzten einfachen A-Scans gewonnen werden, die in einem transveral flächenhaften Bereich (in x- und y-Richtung) verteilt sind. Die oben beschriebenen Mittelungsverfahren an zweidimensionalen Bereichen werden dadurch auf drei Dimensionen erweitert. Ferner kann das in allen Anordnungen erwähnte Dove-Prisma (149, 449 und 549) auch dazu benutzt werden, dreidimensionale OCT Bilder aus dem vorderen (beispielsweise der Vorderkammer) und dem hinteren Augenbereich (beispielsweise der Retina) zu gewinnen. Entsprechende Messdatensätze im dreidimensionalen Raum können entlang Geraden mit unterschiedlichen azimutalen Orientierungen normal zur Augenachse gewonnen werden, wenn man mit Hilfe dieses Prismas das Auge gegenüber dem Interferometer (virtuell) in verschiedene azimutale Orientierungen dreht. Hierzu genügt beispielsweise bei dem DovePrisma eine einfache Drehvorrichtung, die dieses um eine Parallele zur Hypothenusenfläche dreht; das Bild wird hierbei um den doppelten Winkel gedreht. Ähnlich können hierzu auch andere Reversionsprismen, wie das Prisma von Abbe/König, das Prisma von Schmidt/Pechan oder das aus drei Prismen bestehende Prismenumkehrsystem nach Uppendahl verwendet werden.

## Patentansprüche

1. Vorrichtung zur Messung an einem Auge (4), insbesondere zur Messung von Vorderkammertiefe, Linsendicke, Hornhautdicke, Netzhautschichtdicken oder Achslänge, wobei die Vorrichtung ein Interferometer (10) umfaßt, mindestens einen Meßstrahl (7, 8) entlang einer optischen Achse (OA) in das Auge (4) fokussiert, rückgestreute Strahlung aufnimmt und durch Zeitdomänen-, Spektraldomänen- oder Fourierdomänen-Kohärenzreflektrometrie interferometrisch ein Strukturen des Auges anzeigendes Meßsignal erzeugt, eine Verstelleinrichtung (13; 16; 17; 19; 21) zur lateralen und/oder axialen Verschiebung des Fokus im Auge (4) oder zur Variation eines Polarisationszustandes des Meßstrahls (7, 8) und eine Steuereinrichtung (22), die das Interferometer ansteuert, aufweist, **dadurch gekennzeichnet, daß** die Steuereinrichtung (22) aus der rückgestreuten Strahlung mehrere A-Scan-Einzelsignale erzeugt und diese zu einem A-Scan-Meßsignal zusammenfaßt und so ausgebildet ist, daß sie die Verstelleinrichtung (13; 16; 17; 19; 21) zur Verschiebung der Lage des Fokus oder zur Polarisationsvariation während der Aufnahme der rückgestreuten Strahlung, aus der die Steuereinrichtung (22) die A-Scan-Einzelsignale erzeugt, ansteuert, und rückgestreute Strahlung bei mehreren verschiedenen Lagen des Fokus oder mehreren verschiedenen Polarisationszuständen der Meßstrahlung (7, 8) zum A-Scan-Meßsignal beiträgt, wobei zumindest ein Teil des Veränderungsumfangs der Fokuslagen- oder Polarisationszustandsveränderung während der Aufnahme der rückgestreuten Strahlung für eines der mehreren A-Scan-Einzelsignale ausgeführt wird.

2. Vorrichtung nach Anspruch 1, **dadurch gekennzeichnet, daß** die Steuereinrichtung (22) die mehreren A-Scan-Einzelsignale zum A-Scan-Meßsignal zusammenfaßt, ohne dabei die Ansteuerung oder einen Betriebszustand der Verstelleinrichtung (13; 16; 17; 19; 21) zu berücksichtigen.

3. Vorrichtung nach Anspruch 1 oder 2, **dadurch gekennzeichnet, daß** die Steuereinrichtung (22) die Verstelleinrichtung (13; 16; 17; 19) so ansteuert, daß während der Aufnahme der rückgestreuten Strahlung die Lage des Fokus im Auge (4) um mindestens einen halben Fokusdurchmesser wandert.

4. Vorrichtung nach einem der obigen Ansprüche, **dadurch gekennzeichnet, daß** die Steuereinrichtung (22) beim Zusammenfassen der A-Scan-Einzelsignale zum A-Scan-Meßsignal die einzelnen A-Scan-Einzelsignale entsprechend ihres Signalverlaufes selektiert und wichtet.

**5.** Vorrichtung nach einem der obigen Ansprüche, **dadurch gekennzeichnet, daß** zur Vermessung eine mit der Steuereinrichtung (22) verbundene Fixierbildeinrichtung (16) vorgesehen ist, die einem Patienten zur Ausrichtung des Auges (4) ein Fixierbild (12) darbietet, wobei die Fixierbildeinrichtung (16) zur lateralen Verschiebung der Lage des Fokus das dargebotene Fixierbild (12) verschiebt.

**6.** Vorrichtung nach einem der obigen Ansprüche, **dadurch gekennzeichnet, daß** im Strahlengang des Meßstrahls (7, 8) ein angetriebenes, lageverstellbares Optikelement (13), insbesondere eine verschiebbare Linse, vorgesehen ist, dessen Lageverstellung die Lage des Fokus verschiebt.

**7.** Verfahren zur Messung an einem Auge, insbesondere zur Messung von Vorderkammertiefe, Linsendicke, Hornhautdicke, Netzhautschichtdicken oder Achslänge, wobei mindestens ein Meßstrahl entlang einer optischen Achse in das Auge fokussiert, rückgestreute Strahlung aufgenommen und durch Zeitdomänen-, Spektraldomänen- oder Fourierdomänen-Kohärenzreflektrometrie interferometrisch ein Strukturen des Auges anzeigendes Meßsignal erzeugt wird und die Lage des Fokus im Auge lateral und/oder axial verschoben oder ein Polarisationszustand des Meßstrahls variiert wird, **dadurch gekennzeichnet, daß** aus der rückgestreuten Strahlung interferometrisch mehrere A-Scan-Einzelsignale erzeugt und zu einem A-Scan-Meßsignal zusammengefaßt werden, wobei die Verschiebung der Lage des Fokus oder die Variation des Polarisationszustandes während der Aufnahme der rückgestreuten Strahlung, aus der die mehreren A-Scan-Einzelsignale erzeugt werden, ausgeführt wird, und wobei rückgestreute Strahlung bei mehreren verschiedenen Lagen des Fokus oder mehreren verschiedenen Polarisationszuständen der Meßstrahlung zum A-Scan-Meßsignal beiträgt, wobei zumindest ein Teil des Veränderungsumfangs der Fokuslagen- oder Polarisationszustandsveränderung während der Aufnahme der rückgestreuten Strahlung für eines der mehreren A-Scan-Einzelsignale ausgeführt wird.

**8.** Verfahren nach Anspruch 7, **dadurch gekennzeichnet, daß** die mehreren A-Scan-Einzelsignale zum A-Scan-Meßsignal zusammengefaßt werden, ohne daß dabei die
Verschiebung der Lage des Fokus bzw. die Variation des Polarisationszustandes berücksichtigt wird.

**9.** Verfahren nach Anspruch 7 oder 8, **dadurch gekennzeichnet, daß** die Lage des Fokus während der Aufnahme der rückgestreuten Strahlung um mindestens einen halben Fokusdurchmesser verschoben wird.

**10.** Verfahren nach einem der obigen Verfahrensansprüche, **dadurch gekennzeichnet, daß** beim Zusammenfassen der A-Scan-Einzelsignale zum A-Scan-Meßsignal die einzelnen A-Scan-Einzelsignale entsprechend ihres Signalverlaufes selektiert und gewichtet werden.

**11.** Vorrichtung oder Verfahren nach einem der obigen Ansprüche, **dadurch gekennzeichnet, daß** die laterale Verschiebung der Lage des Fokus mit einer Verschiebegeschwindigkeit erfolgt, die kleiner ist als der Quotient aus dem halben Fokusdurchmesser und einer Aufnahmedauer der Strahlung für ein A-Scan-Einzelsignal.

**12.** Verfahren nach einem der obigen Verfahrensansprüche, **dadurch gekennzeichnet, daß** aus den A-Scan-Einzelsignalen oder mehreren A-Scan-Meßsignalen die Dicke einer Augenlinse des Auges ermittelt wird, indem für alle Signale die Positionen der Linsenvorderfläche und -rückfläche ermittelt werden, und die Differenz zwischen der am meisten anterior liegenden der ermittelten Positionen der Linsenvorderfläche und der am meisten posterior liegenden der ermittelten Positionen der Linsenrückflächen berechnet und als Dicke der Augenlinse genommen wird.

**13.** Verfahren nach einem der obigen Verfahrensansprüche, **dadurch gekennzeichnet, daß** zur Bestimmung von Parametern einer Augenlinse des Auges Paare aus ermittelter Position einer Linsenvorderfläche und ermittelter Position einer Linsenrückfläche in einem Diagramm aufgetragen werden, wobei entlang einer Diagramm-Achse die Position der Linsenvorderfläche und entlang der anderen Diagramm-Achse die Position der Linsenrückfläche aufgetragen werden, die erhaltenen Punkte zu einer Kurve verbunden oder interpoliert werden, und die Form der Kurve hinsichtlich der Lage und Form der Augenlinse ausgewertet wird.

**14.** Verfahren nach Anspruch 7, **dadurch gekennzeichnet, daß** das A-Scan-Meßsignal als Summe aus mehreren seitlich zueinander versetzten einfachen A-Scan-Einzelsignalen gebildet wird und eine Distanzmessung am Auge mittels optischer Kwthahärent -Interferometrie (KKI) durch Darstellung der Grenzflächen der zu messenden Abstände als Kurz-Kohärenz Interferogramm im A-Scan-Meßsignal erfolgt.

**15.** Verfahren nach Anspruch 7, **dadurch gekennzeichnet, daß** das A-Scan-Meßsignal als Summe aus mehreren A-Scan-Einzelsignalen aus bestimmten transversalen Positionen im Auge sowie zu bestimmten Zeitpunkten innerhalb der Herzpulsperiode gebildet wird und eine Distanzmessung am Auge mittels optischer Kwthahärent -Interferometrie (KKI) durch Darstellung der Grenzflächen der zu messenden Abstände als Kurz-Kohärenz Interferogramm im A-Scan-Meßsignal erfolgt.

**Claims**

1.  Device for measurement on an eye (4), in particular for measuring anterior chamber depth, lens thickness, corneal thickness, retinal layer thicknesses or axial length, wherein the device comprises an interferometer (10), focuses at least one measuring beam (7, 8) along an optical axis (OA) into the eye (4), records backscattered radiation and, by time domain, spectral domain or Fourier domain coherence reflectometry, interferometrically generates a measurement signal which indicates structures of the eye, has an adjustment apparatus (13; 16; 17; 19; 21) for lateral and/or axial displacement of the focus in the eye (4) or for variation of a polarisation status of the measuring beam (7, 8) and a control apparatus (22) which actuates the interferometer, **characterised in that** the control apparatus (22) generates a plurality of A-scan individual signals from the backscattered radiation and combines these to form an A-scan measurement signal and is thus formed in such a manner that it actuates the adjustment apparatus (13; 16; 17; 19; 21) for displacement of the position of the focus or for polarisation variation during recording of the backscattered radiation, from which backscattered radiation the control apparatus (22) generates the A-scan individual signals, and backscattered radiation in the case of several different positions of the focus or several different polarisation statuses of the measurement radiation (7, 8) contributes to the A-scan measurement signal, wherein at least part of the scope of change of the change in focal positions or polarisation status is performed during recording of the backscattered radiation for one of the plurality of A-scan individual signals.

2.  Device according to Claim 1, **characterised in that** the control apparatus (22) combines the plurality of A-scan individual signals to form the A-scan measurement signal without in this case taking into account the actuation or an operating status of the adjustment apparatus (13; 16; 17; 19; 21).

3.  Device according to Claim 1 or 2, **characterised in that** the control apparatus (22) actuates the adjustment apparatus (13; 16; 17; 19) in such a manner that, during the recording of the backscattered radiation, the position of the focus in the eye (4) migrates by at least half a focus diameter.

4.  Device according to one of the above claims, **characterised in that** the control apparatus (22) selects and weights the individual A-scan individual signals in accordance with their signal profile when combining the A-scan individual signals to form the A-scan measurement signal.

5.  Device according to one of the above claims, **characterised in that**, for the purpose of measurement, a fixing image apparatus (16) connected to the control apparatus (22) is provided which provides a patient with a fixing image (12) for alignment of the eye (4), wherein the fixing image apparatus (16) displaces the provided fixing image (12) for lateral displacement of the position of the focus.

6.  Device according to one of the above claims, **characterised in that** a driven, position-adjustable optical element (13), in particular a displaceable lens, is provided, the position adjustment of which displaces the position of the focus, in the beam path of the measuring beam (7, 8).

7.  Method for measurement on an eye, in particular for measuring anterior chamber depth, lens thickness, corneal thickness, retinal layer thicknesses or axial length, wherein at least one measuring beam along an optical axis is focused into the eye, backscattered radiation is recorded and, by time domain, spectral domain or Fourier domain coherence reflectometry, a measurement signal which indicates structures of the eye is interferometrically generated and the position of the focus in the eye is laterally and/or axial displaced or a polarisation status of the measuring beam is varied, **characterised in that** a plurality of A-scan individual signals are interferometrically generated from the backscattered radiation and combined to form an A-scan measurement signal, wherein the displacement of the position of the focus or the variation of the polarisation status is performed during recording of the backscattered radiation, from which backscattered radiation the plurality of A-scan individual signals are generated, and wherein backscattered radiation in the case of several different positions of the focus or several different polarisation statuses of the measurement radiation contributes to the A-scan measurement signal, wherein at least part of the scope of change of the change in focal position or polarisation status is performed during recording of the backscattered radiation for one of the plurality of A-scan individual signals.

8. Method according to Claim 7, **characterised in that** the plurality of A-scan individual signals are combined to form the A-scan measurement signal without in this case taking into account the displacement of the position of the focus or the variation of the polarisation status.

9. Method according to Claim 7 or 8, **characterised in that**, during the recording of the backscattered radiation, the position of the focus is displaced by at least half a focus diameter.

10. Method according to one of the above method claims, **characterised in that** the individual A-scan individual signals are selected and weighted in accordance with their signal profile when combining the A-scan individual signals to form the A-scan measurement signal.

11. Device or method according to one of the above claims, **characterised in that** the lateral displacement of the position of the focus is performed with a displacement speed which is lower than the quotient from half the focus diameter and a recording duration of the radiation for an A-scan individual signal.

12. Method according to one of the above method claims, **characterised in that** the thickness of an eye lens of the eye is identified from the A-scan individual signals or plurality of A-scan measurement signals **in that** the positions of the front surface and rear surface of the lens are identified for all the signals, and the difference between the most anterior of the identified positions of the front surface of the lens and the most posterior of the identified positions of the rear surface of the lens is calculated and is taken as the thickness of the eye lens.

13. Method according to one of the above method claims, **characterised in that**, in order to determine parameters of an eye lens of the eye, pairs from an identified position of a front surface of a lens and an identified position of a rear surface of the lens are plotted in a diagram, wherein the position of the front surface of the lens is plotted along one diagram axis and the position of the rear surface of the lens is plotted along the other diagram axis, the obtained points are connected or interpolated to form a curve, and the shape of the curve is evaluated in terms of the position and shape of the eye lens.

14. Method according to Claim 7, **characterised in that** the A-scan measurement signal is formed as a sum of a plurality of single A-scan individual signals which are laterally offset with respect to one another and a distance measurement is performed on the eye by means of optical short coherence interferometry by mapping of the boundaries of the distances to be measured as a short coherence interferogram in the A-scan measurement signal.

15. Method according to Claim 7, **characterised in that** the A-scan measurement signal is formed as a sum of a plurality of A-scan individual signals from specific transversal positions in the eye and at specific points in time within the heart rate period and a distance measurement is performed on the eye by means of optical short coherence interferometry by mapping of the boundaries of the distances to be measured as a short coherence interferogram in the A-scan measurement signal.

**Revendications**

1. Dispositif pour la mesure dans un oeil (4), en particulier pour mesurer la profondeur de la chambre antérieure, l'épaisseur du cristallin, l'épaisseur de la cornée, l'épaisseur des couches de la rétine ou la longueur d'axe, dans lequel le dispositif comprend un interféromètre (10), il focalise au moins un rayon de mesure (7, 8) le long d'un axe optique (AO) dans l'oeil (4), il absorbe le rayonnement rétrodiffusé et il produit un signal de mesure indiquant des structures de l'oeil de façon interférométrique par réflectométrie cohérente à domaine de temps, à domaine spectral ou à domaine de Fourier et il possède un dispositif de réglage (13 ; 16 ; 17 ; 19 ; 21) pour le déplacement latéral et/ou axial du point focal dans l'oeil (4) ou pour la modification d'un état de polarisation du rayon de mesure (7, 8) et un dispositif de commande (22) qui commande l'interféromètre, **caractérisé en ce que** le dispositif de commande (22) produit, à partir du rayonnement rétrodiffusé, plusieurs signaux individuels A-scan et les rassemble pour obtenir un signal de mesure A-scan et il est réalisé de telle sorte qu'il commande le dispositif de réglage (13 ; 16 ; 17 ; 19 ; 21) en vue du déplacement de la position du point focal ou en vue de la modification de la polarisation pendant l'absorption du rayonnement rétrodiffusé à partir duquel le dispositif de commande (22) produit les signaux individuels A-scan et **en ce que** le rayonnement rétrodiffusé contribue, avec plusieurs positions différentes du point focal ou plusieurs états de polarisation différents du rayonnement de mesure (7, 8), au signal de mesure A-scan, étant entendu qu'au moins une partie du degré de modification de la position du point focal ou de l'état de la polarisation est exécutée pendant l'absorption du rayonnement rétrodiffusé pour l'un des multiples signaux individuels A-scan.

**2.** Dispositif selon la revendication 1, **caractérisé en ce que** le dispositif de commande (22) rassemble les multiples signaux individuels A-scan pour obtenir le signal de mesure A-scan sans prendre en considération la commande ou un état de fonctionnement du dispositif de réglage (13 ; 16 ; 17; 19; 21).

**3.** Dispositif selon la revendication 1 ou 2, **caractérisé en ce que** le dispositif de commande (22) commande le dispositif de réglage (13; 16; 17; 19) de telle sorte que pendant l'absorption du rayonnement rétrodiffusé, la position du point focal dans l'oeil (4) se déplace sur au moins une moitié du diamètre du point focal.

**4.** Dispositif selon l'une des revendications précédentes, **caractérisé en ce que** le dispositif de commande (22), lorsqu'il rassemble les signaux individuels A-scan pour obtenir le signal de mesure A-scan, sélectionne et pondère les signaux individuels A-scan en fonction de leur tracé de signal.

**5.** Dispositif selon l'une des revendications précédentes, **caractérisé en ce qu'**il est prévu, en vue de la mesure, un dispositif d'image à fixer (16) relié au dispositif de commande (22) qui présente à un patient une image à fixer (12) en vue de l'orientation de l'oeil (4), étant entendu que le dispositif d'image à fixer (16) déplace l'image à fixer (12) présentée en vue du déplacement latéral de la position du point focal.

**6.** Dispositif selon l'une des revendications précédentes, **caractérisé en ce qu'**il est prévu, dans le trajet de rayonnement du rayon de mesure (7, 8), un élément optique entraîné à position réglable (13), en particulier une lentille déplaçable, dont le réglage de la position déplace la position du point focal.

**7.** Procédé pour la mesure dans un oeil (4), en particulier pour mesurer la profondeur de la chambre antérieure, l'épaisseur du cristallin, l'épaisseur de la cornée, l'épaisseur des couches de la rétine ou la longueur d'axe, dans lequel au moins un rayon de mesure est focalisé le long d'un axe optique (AO) dans l'oeil (4), le rayonnement rétrodiffusé est absorbé et un signal de mesure indiquant des structures de l'oeil est produit de façon interférométrique par réflectométrie cohérente à domaine de temps, à domaine spectral ou à domaine de Fourier et la position du point focal dans l'oeil est déplacée dans le sens latéral et/ou axial ou un état de polarisation du rayon de mesure est modifié, **caractérisé en ce qu'**à partir du rayonnement rétrodiffusé, plusieurs signaux individuels A-scan sont produits de façon interférométrique et rassemblés pour obtenir un signal de mesure A-scan, étant entendu que le déplacement de la position du point focal ou la modification de l'état de la polarisation sont exécutés pendant l'absorption du rayonnement rétrodiffusé à partir duquel les multiples signaux individuels A-scan sont produits, que le rayonnement rétrodiffusé contribue, avec plusieurs positions différentes du point focal ou plusieurs états de polarisation différents du rayonnement de mesure, au signal de mesure A-scan, et qu'au moins une partie du degré de modification de la position du point focal ou de l'état de la polarisation est exécutée pendant l'absorption du rayonnement rétrodiffusé pour l'un des multiples signaux individuels A-scan.

**8.** Procédé selon la revendication 7, **caractérisé en ce que** les multiples signaux individuels A-scan sont rassemblés pour obtenir le signal de mesure A-scan sans que le déplacement de la position du point focal ou la modification de l'état de la polarisation soient pris en considération.

**9.** Procédé selon la revendication 7 ou 8, **caractérisé en ce que** la position du point focal est déplacé sur au moins une moitié du diamètre du point focal pendant l'absorption du rayonnement rétrodiffusé.

**10.** Procédé selon l'une des revendications de procédé précédentes, **caractérisé en ce que**, lorsque les signaux individuels A-scan sont rassemblés pour obtenir le signal de mesure A-scan, les signaux individuels A-scan sont sélectionnés et pondérés en fonction de leur tracé de signal.

**11.** Procédé selon l'une des revendications de procédé précédentes, **caractérisé en ce que** le déplacement latéral de la position du point focal est réalisée à une vitesse de déplacement qui est inférieure au quotient entre la moitié du diamètre du point focal et une durée d'absorption du rayonnement pour un signal individuel A-scan.

**12.** Procédé selon l'une des revendications de procédé précédentes, **caractérisé en ce que** l'épaisseur d'un cristallin d'un oeil est calculée à partir des signaux individuels A-scan ou de plusieurs signaux de mesure A-scan en ce que pour tous les signaux, les positions de la face avant et de la face arrière du cristallin sont déterminées et la différence entre la plus antérieure des positions déterminées sur la face avant du cristallin et la plus postérieure des positions déterminées de la face arrière du cristallin est calculée et admise comme l'épaisseur du cristallin.

**13.** Procédé selon l'une des revendications de procédé précédentes, **caractérisé en ce qu'**en vue de déterminer les paramètres d'un cristallin de l'oeil, des paires de la position déterminée d'une face avant du cristallin et de la position déterminée d'une face arrière du cristallin sont inscrites dans un graphique, étant entendu que la position de la face avant du cristallin est inscrite sur un axe du graphique et la position de la face arrière du cristallin sur l'autre axe du graphique, les points obtenus sont reliés ou interpolés pour obtenir une courbe et la forme de la courbe est évaluée par rapport à la position et à la forme du cristallin.

14. Procédé selon la revendication 7, **caractérisé en ce que** le signal de mesure A-scan est formé comme la somme de plusieurs signaux individuels A-scan simples décalés latéralement les uns par rapport aux autres et une mesure de distance est réalisée dans l'oeil au moyen d'une interférométrie cohérente courte (ICC) par la représentation des surfaces limites des distances à mesurer en tant qu'interférogramme cohérent court dans le signal de mesure A-scan.

15. Procédé selon la revendication 7, **caractérisé en ce que** le signal de mesure A-scan est formé comme la somme de plusieurs signaux individuels A-scan provenant de positions transversales déterminées dans l'oeil ainsi qu'à des moments déterminés pendant la période de pulsation cardiaque et une mesure de distance est réalisée dans l'oeil au moyen d'une interférométrie cohérente courte (ICC) par la représentation des surfaces limites des distances à mesurer en tant qu'interférogramme cohérent court dans le signal de mesure A-scan.

Fig. 1

Fig. 2

Fig. 3

Fig. 4a

Fig. 5

Fig. 6

Fig. 7

Fig. 8

Fig. 9

Fig. 10

Fig. 11

Fig. 12

Fig. 4b

Fig. 13

Fig. 14

Fig. 15

Fig. 16

Fig. 17

Fig. 18

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20060109477 A1
- US 7330270 B2
- WO 2007065670 A
- WO 2007100935 A2

### In der Beschreibung aufgeführte Nicht-Patentliteratur

- **LEXER et al.** Wavelength-tuning interferometry of intraocular distances. *APPLIED OPTICS,* vol. 36 (25
- **T. M. JORGENSEN ; L. THRANE ; M. MOGENSEN ; F. PEDERSEN ; P. E. ANDERSEN.** Speckle reduction in optical coherence tomography images of human skin by a spatial diversity method. *Proc. of SPIE-OSA Biomedical Optics,* 2007, vol. 6627
- Polarization Effects in Optical Coherence Tomography of Various Biological Tissues. **JOHANNES F. DE BOER et al.** IEEE Journal of Selected Topics in Quantum Electronics. IEEE Service Center, 01. August 1999, vol. 5
- **J. M. SCHMITT.** Optical Coherence Tomography (OCT): A Review. *IEEE Selected Topics in Quantum Electronics,* 1999, vol. 5 (4), 1205-1215
- **FERCHER et al.** Measurement of Intraocular Distances by Backscattering Spectral Interferometry. *Opt. Comm.,* vol. 117, 43